# EUROPEAN PATENT SPECIFICATION

(11) **EP 2 630 859 B1**
(45) Date of publication and mention of the grant of the patent: **22.02.2017**
(21) Application number: 12305223.5
(22) Date of filing: 24.02.2012
(51) Int. Cl.: A01G 1/04, A01H 17/00

(54) **Reforestation of a soil area with co culture of tree species and nurse plants**
Wiederaufforstung eines Bodenbereichs mit Co-Anpflanzung von Baumarten und Ammenpflanzen
Reforestation d'une zone de sol avec la coculture d'espèces d'arbres et de plantes compagnes

(43) Date of publication of application: 28.08.2013
(73) Proprietor: Institut de Recherche pour le Developpement (I.R.D.), 13572 Marseille Cedex 02 (FR); Centre de Coopération Internationale en Recherche Agronomique pour le Développement, 75116 Paris 16ème (FR); Centre National de Recherches sur L'Environnement (CNRE), Antananarivo, Fiadanana (MG)
(72) Inventor: Duponnois, Robin, F-71640 Mercurey (FR); Baohanta, Rondro Harinisainana, 101 Antananarivo (MG); Ramanankierana, Heriniaina, 101 Morarano-Antananarivo (MG); Prin, Yves, 34000 Montpellier (FR)
(74) Representative: Grosset-Fournier, Chantal Catherine

(56) References cited:
- EP-A1- 0 521 758
- DUPONNOIS R ET AL: "Monitoring the Development of Nurse Plant Species to Improve the Performances of Reforestation Programs in Mediterranean Areas", MICROBIAL STRATEGIES FOR CROP IMPROVEMENT SPRINGER, 233 SPRING STREET, NEW YORK, NY 10013, UNITED STATES, 2009, pages 255-265, XP002680549,
- DUPONNOIS R ET AL: "Nurse shrubs increased the early growth of Cupressus seedlings by enhancing belowground mutualism and soil microbial activity", SOIL BIOLOGY & BIOCHEMISTRY, vol. 43, no. 10, October 2011 (2011-10), pages 2160-2168, XP002680550, ISSN: 0038-0717
- OUAHMANE L ET AL: "Lavandula species as accompanying plants in Cupressus replanting strategies: Effect on plant growth, mycorrhizal soil infectivity and soil microbial catabolic diversity", APPLIED SOIL ECOLOGY, ELSEVIER, AMSTERDAM, NL, vol. 34, no. 2-3, 1 December 2006 (2006-12-01), pages 190-199, XP027997262, ISSN: 0929-1393 [retrieved on 2006-12-01]
- NAINA RAMANANKIERANA ET AL: "Arbuscular mycorrhizas and ectomycorrhizas of Uapaca bojeri L. (Euphorbiaceae): sporophore diversity, patterns of root colonization, and effects on seedling growth and soil microbial catabolic diversity", MYCORRHIZA, SPRINGER, BERLIN, DE, vol. 17, no. 3, 13 January 2007 (2007-01-13), pages 195-208, XP019518859, ISSN: 1432-1890, DOI: 10.1007/S00572-006-0095-0
- KISA MARIJA ET AL: "Arbuscular mycorrhizal symbiosis can counterbalance the negative influence of the exotic tree species Eucalyptus camaldulensis on the structure and functioning of soil microbial communities in a sahelian soil", FEMS MICROBIOLOGY ECOLOGY, vol. 62, no. 1, October 2007 (2007-10), pages 32-44, XP002680551, ISSN: 0168-6496
- BILGO ABLASSE ET AL: "Response of native soil microbial functions to the controlled mycorrhization of an exotic tree legume, Acacia holosericea in a Sahelian ecosystem", MYCORRHIZA, vol. 22, no. 3, April 2012 (2012-04), pages 175-187 URL, XP002680552,
- BA AMADOU M ET AL: "Ectomycorrhizal symbiosis of tropical African trees", MYCORRHIZA, vol. 22, no. 1, January 2012 (2012-01), pages 1-29 URL, XP002680553,
- KROPP B R ET AL: "ECTOMYCORRHIZAE IN REFORESTATION", CANADIAN JOURNAL OF FOREST RESEARCH, vol. 20, no. 4, 1990, pages 438-451, XP002680554, ISSN: 0045-5067
- CARRILLO-GARCIA A ET AL: "Nurse plants, mycorrhizae, and plant establishment in a disturbed area of the Sonoran Desert", RESTORATION ECOLOGY, vol. 7, no. 4, December 1999 (1999-12), pages 321-335, XP002680555, ISSN: 1061-2971
- MARK G L ET AL: "In vitro culture of arbuscular mycorrhizal fungus and Frankia for inoculation of micropropagated Casuarina equisetifolia L", CANADIAN JOURNAL OF BOTANY / JOURNAL CANADIEN DE BOTANIQUE, NATIONAL RESEARCH COUNCIL, OTTAWA, CA, vol. 77, no. 9, 1 September 1999 (1999-09-01), pages 1391-1397, XP008031127, ISSN: 0008-4026, DOI: 10.1139/CJB-77-9-1391
- BAOHANTA R. ET AL.: "Restoring native forest ecosystems after exotic tree plantation in Madagascar: combination of the local ectotrophic species Leptolena bojeriana and Uapaca bojeri mitigates the negative influence of the exotic species Eucalyptus camaldulensis and Pinus patula", BIOLOGICAL INVASIONS, 11 May 2012 (2012-05-11), XP002680556,

## Description

The present invention relates to the reforestation of a soil area with co culture of tree species with nurse plants.

Numerous agricultural practices lead to soil degradations and losses of biodiversity in tropical areas. These anthropogenic impacts do not only degrade natural plant communities (population structure and species diversity) but also physico-chemical and biological soil properties (nutrient availability, microbial activity, soil structure, etc) (Styger et al. 2007, Influence of slash-and-burn farming practices on fallow succession and land degradation in the rainforest region of Madagascar. Agr Ecosyst Environ 119: 257-269). In order to reverse this loss of fertility and to limit soil erosion, some revegetation programs have been undertaken in Madagascar using fast-growing exotic trees. Reforestation with eucalyptus (*E. robusta, E. rostrata, E. camaldulensis*) and later pine (*P. khesya, P. patula*) provided a wood supply mainly for the cooking fires in the region but also for construction timbers, casketts, etc (Gade 1996, Deforestation and its effects in highland Madagascar. Moutain Res Dev 16: 101-116). By the 1930s, plantations have been set out by local communities, institutions and individuals (Parrot 1925, Le reboisement de Madagascar par le moyen des forêts de "fokon olona". Bulletin Economique (Antananarivo), 1-2 (supp.): 55-57). However exotic trees can threaten ecosystems or habitats by altering ecological interactions among native plants in the introduction zone (Rejmanek 2000; Invasive plants: approaches and predictions. Aust Ecol 25: 497-506; Callaway and Ridenour 2004, Novel weapons: invasive success and the evolution of increased competitive ability. Front Ecol Environ 2: 436-443) that could compromise their role in sustainable development. Exotic speciess can act directly on native plant communities by allelopathic effects or by higher performance in an introduction site that influence vegetation dynamics, community structure and composition (del Moral and Muller 1970, The allelopathic effects of Eucalyptus camaldulensis. Am Midland Nat 83: 254-282; Thébaud and Simberloff 2001, Are plants really larger in their introduced ranges? Am Nat 157: 231-236). They also can alter biochemical cycling compared to native plants (Ashton et al. 2005, Invasive species accelerate decomposition and litter nitrogen loss in a mixed deciduous forest. Ecol Appl 15: 1263-1272).

Numerous studies have shown that ectomycorrhizal (ECM) vegetation is highly dependent on ECM fungi for their growth and survival (Smith and Read 2008, Mycorrhizal symbiosis, 3rd ed. Academic Press, 800 p.). Limitation of the presence, abundance and community composition of ectomycorrhizal fungi can result from natural (Terwilliger and Pastor 1999, Small mammals, ectomycorrhizae, and conifer succession in beaver meadows. Oikos 85: 83-94) or anthropogenic disturbance (Jones et al. 2003, Ectomycorrhizal fungal communities in young forest stands regenerating after clearcut logging. New Phytol 157: 399-422) and the lack of established ectomycorrhizal fungi in soils may limit the establishment or re-establishment of ECM tree species seedlings (Marx 1991, The practical significance of ectomycorrhizae in forest establishment. Ecophysiology of Ectomycorrhizaeof Forest Trees, Marcus Wallenberg Foundation Symposia proceedings 7: 54-90).

One of the aims of the invention is to provide a simple way for reforesting a soil unadapted to the good growth of a tree species by means of a nurse plant liable to counterbalance the negative impacts of for example exotic trees cultured in said soil.

Another aim of the invention is to provide a process of reforestation of a soil based on the co culture of a tree species with a nurse plant.

Another aim of the invention is to take advangtage of the aerial parts of said nurse plant.

The present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area, for promoting the growth of at least one seed or at least one plant of said tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture,
the ectomycorrhizal colonization C1 of the roots of said tree species cultured in said soil area, before said pre culture, being decreased by a factor comprised from about 2 to about 5 compared with the ectomycorrhizal colonization obtained in a control soil comprising said tree species, in particular due to the growth of an exotic species previously cultured in said soil area before said pre culture, said ectomycorrhizal colonization C1 comprising:
the presence of at least one ectomycorrhizal fungus, in particular of an exotic species, unfavorable to the growth of said particular tree species in said soil area in a relative ratio of at least 10%, and/or
the presence of at least one essential ectomycorrhizal fungus to promote the growth of said particular tree species in said soil area in a relative ratio equal or less than 0.1%,
the ectomycorrhizal colonization C2 of the roots of said tree species being, after said pre culture of said nurse plant and 5 months of culture of said tree species, in particular in a soil area having a volume of approximately 2,5L, recovered in a range from about 70% to about 100%, and said ectomycorrhizal colonization C2 comprising:
at least one unfavorable ectomycorrhizal fungus, present in said soil area before said pre culture, in a relative ratio equal or less than 0.1% and /or
the presence of at least one recovered essential ectomycorrhizal fungus in a relative ratio of about at least 10%.

The inventors have found in an unexpected manner in the present invention that the identification and selection of a nurse plant based on the determination of its ectomycorrhizal colonization and its compatibility with a tree species, and the pre culture of said nurse plant in a soil area, wherein an exotic species or a plant having the same negative or detrimental impact on the growth of a tree species as an exotic species has been cultured, were able to:
- increase the content of the ectomycorrhizal colonization of the roots of the tree species before said pre culture in order to recover partially or totally the ectomycorrhizal colonization of said tree species observed in a soil wherein no exotic species or a plant having the same negative or detrimental impact on the growth of a tree species has been cultured,
- substantially eliminate (relative ratio less than 0.1%) at least one unfavorable ectomycorrhizal fungus,
- recover (relative ratio of about at least 10%) at least one essential ectomycorrhizal fungus.

Thus the inventors have made the link between the mycorrhizal colonization of a selected nurse plant and the benefic effects of said mycorrhizal colonization on the growth tree species as well as the ability of said mycorrhizal colonization to stop the presence of unfavorable ectomycorrhizal fungi and develop the presence of essential ectomycorrhizal fungi, giving in fact the reversing of unfavorable ectomycorrhizal fungi to essential ectomycorrhizal fungi.

In other words, the pre culture of an identified nurse plant the ectomycorrhizal colonization of which being compatible with the one of a tree species is liable to reverse the negative or detrimental impacts of exotic species or other plants observed on the growth a tree species.

By the term "nurse plant" is meant an accompanying plant that is usually found in a soil area wherein a tree species is cultured.

The ectomycorrhizal fungi of the roots of the nurse plant that is located in the soil area to reforest are previously defined and their compatibility with those borne by the tree species is analyzed allowing identifying and selecting the nurse plant bearing at least one fungus essential to the growth of the tree species and preferably the largest number of essential fungi and more preferably the lowest number of unfavorable fungi.

Defining the ectomycorrhizal fungi of the nurse plant and determining the one of the tree species can be made in any order but both must be made before the selection of the nurse plant.

The identification of said ectomycorrhizal colonization and the determination of the relative content of each ectomycorrhizal fungus can be carried out with methods well known from a man skilled in the art.

In particular, said identification and determination is carried out by the internal transcribed spacers sequence of the restriction fragment length polymorphism types.

Thus several nurse plants can be found in the soil area wherein a tree species is cultured and the analysis of the ectomycorrhizal colonization of the roots of said nurse plants allows selecting the best nurse plant based on the compatibility of the ectomycorrhizal colonization with the one of the tree species cultured in said soil area.

By "ectomycorrhizal colonization" is meant the rate of colonization by at least one ectomycorrhizal fungus of the total root length (Duponnois & Garbaye 1991, Techniques for controlled synthesis of the Douglas-fir - Laccaria laccata ectomycorrhizal symbiosis. Ann For Sci: 641-650)

The nurse plant can be a seed of said plant but also a semence, a grain, a fruit, a pip, a pit or the plant itself.

The nurse plant is "submitted to a pre culture" means that a seed, a semence, a grain, a fruit, a pip, or a pit is introduced in said soil area and grown until it becomes a plant and then further grown from 3 to 6 months, in particular 4 months, or the nurse plant itself is introduced in said soil and grown from 3 to 6 months, in particular 4 months.

Below 3 months of pre culture, the nurse plant growth is not enough to contain sufficient ectomycorrhizal colonization of its roots.

Above 6 months, the plant becomes too large to be used.

At least one nurse plant is present in said soil area, that means that one, two, three, four or five or even more seeds of a nurse plant or nurse plants are introduced in said soil area.

The terms "seed" or "plant" of a tree species have the same meaning as above. Thus, that means that one, two, three, four or five or even more seed or plant of a tree species can be introduced in said soil area after pre culture of said nurse plant.

In an advantageous embodiment, only one seed or plant of a tree species is introduced in said soil area, in particular in a soil area having a volume of approximately 2,5L.

The expression "for promoting the growth..." means that the pre culture of the nurse plant allows increasing the growth of the tree species, in particular the shoot biomass and or the root biomass of the tree species cultured in said soil compared to the same tree species cultured in the same soil without pre culture of said nurse plant (Ouahmane et al. 2006, Some Mediterranean plant species (Lavandula spp. and Thymus satureioides) act as potential 'plant nurses' for the early growth of Cupressus atlantica. Plant Ecol 185: 123-134).

The expression "...modifying the ectomycorrhizal colonization of the roots of said tree species in said soil area" means that the pre culture of the nurse plant allows recovering at least one essential fungus for the growth of said tree species in said soil area and to substantially eliminate at least one unfavorable ectomycorrhizal fungus present in said soil area before said pre culture due to the growth of an exotic species previously cultured in said soil area.

The expression "tree species" means a tree liable to grow and usually grown in a soil area to reforest.

After said pre culture of said plant, aerial parts of said nurse plant are cut or not before introducing a seed or a plant of a tree species in said soil area.

By "exotic species" is meant a species that has been introduced from another country, i.e. a species that is not native to the place where it is found.

The expression "the presence of at least one recovered essential ectomycorrhizal fungus" means that at least one of the esseential ectomycorrhizal fungi that were preent in a relative ratio equal or less than 0.1% (C1) is recovered and present in a relative ratio of about at least 10%.

Thus, a tree species cultured in a soil wherein said tree species has never been cultured or in a soil wherein exotic species have been introduced will have an ectomycorrhizal colonization decreased compared with the one of said tree species in a soil wherein said tree species has already been cultured.

Said decrease of ectomycorrhizal colonization is constituted of:
on one hand the presence of at least one ectomycorrhizal fungus, coming from an exotic species or not, unfavorable to the growth of said particular tree species, i.e. an ectomycorrhizal fungi, the presence of which slows down and/or prevent partially the growth of said tree specie, in particular the shoot biomass and/or the root biomass, and/or
on the other hand, the substantial lack of at least one ectomycorrhizal fungus liable to promote the growth of said tree species, i. e. an ectomycorrhizal fungus usually found with the roots of said tree species in a soil where said tree species is usually cultured, in the absence of exotic species or other plants having the same negative or detrimental impact on the growth of tree species.

The identification of said ectomycorrhizal colonization and the evaluation of the relative content of each ectomycorrhizal fungus can be carried out with methods well known from a man skilled in the art.

In particular, said identification and evaluation is carried out by the internal transcribed spacers sequence of the restriction fragment length polymorphism types.

In a preferred embodiment, the mycorrhizal colonization of said nurse plant observed after pre culture comes only from the interaction between the soil area and said nurse plant and:
no exogenous source of ectomycorrhizal fungi (or endomycorrhizal fungi) such as a pre or co inoculation of the seed of the nurse plant, or the nurse plant, has been carried, and
no introduction or amendment of an ectomycorrhizal fungus (or endomycorrhizal fungus) has been carried out into the soil area in an exogenous manner and/or said tree species is cultured without co-culture of a plant other than said nurse plant.

In this embodiment, in the soil area, only the tree species and the nurse plant can be present.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of said tree species and modifying the ectomycorrhizal colonization of the roots of said tree species in said soil area, said pre culture lasting approximately from 3 to 6 months, in particular approximately 4 months, to give a pre cultured plant, as defined above,
wherein said aerial parts of said pre cultured nurse plant are uncut after pre culture of said nurse plant.

In this embodiment, said tree species is introduced into the soil area to reforest after the pre culture of said nurse plant with the totality of said nurse plant. The tree species is therefore co cultured with the nurse plant after said pre culture.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, as defined above,
wherein said aerial parts of said pre cultured nurse plant are cut after pre culture of said nurse plant.

In this embodiment, said tree species is introduced into the soil area to reforest after the pre culture of said nurse plant with only the roots of said nurse plant, as shoot, i.e. leaves and stem have been cut. Thus said nurse plant has not been uprooted but only cut providing the advantage to leave the mycorrhizal colonization of the root nurse plant available only for the tree species and no more for the nurse plant itself.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, as defined above,
wherein said aerial parts of said cut nurse plant are used to produce essential oil or flavor, in function of the pre cultured nurse plant.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, as defined above,
wherein only one seed of a nurse plant or only one nurse plant is pre cultured.

Thus, in this embodiment, one seed of a nurse plant or one nurse plant is introduced in said soil area but the number of seed or plant of a tree species introduced can be one, two, three, four or five or even more.

In an advantageous embodiment, one seed of a nurse plant or one nurse plant is introduced in said soil area and one seed or one plant of a tree species is introduced after pre culture.

In an advantageous embodiment, the increase of the shoot biomass of a tree species after pre culture of only one seed of a nurse plant or only one nurse plant, and 5 months of culture of said tree species, without cutting of the aerial parts of said pre cultured plant nurse after said pre culture, is comprised from approximately 2 to approximately 3.

Thus, one of the advantages of the present invention is that the use of only one seed or plant of a nurse plant pre cultured in a soil area wherein an exotic species or a plant having the same negative or detrimental impact on the growth of a tree species as an exotic species has been cultured, the aerial parts of said pre cultured nurse plant being uncut,
is liable to significantly increase the shoot biomass of a cultured tree species in said soil, compared with the shoot biomass of a cultured tree species in said soil without said pre culture.

In an advantageous embodiment, the increase of the root biomass of a tree species after pre culture of only one seed of a nurse plant or only one nurse plant, and 5 months of culture of said tree species, without cutting of the aerial parts of said pre cultured plant nurse after said pre culture, is comprised from approximately 1.2 to approximately 4.

Another advantage of the present invention is that the use of only one seed or plant of a nurse plant pre cultured in a soil area wherein an exotic species or a plant having the same negative or detrimental impact on the growth of a tree species as an exotic species has been cultured, the aerial parts of said pre cultured nurse plant being uncut, is liable to significantly increase the root biomass of a cultured tree species in said soil, compared with the shoot biomass of a cultured tree species in said soil without said pre culture.

In an advantageous embodiment, the increase of the shoot biomass of a tree species after pre culture of only one seed of a nurse plant or only one nurse plant, and 5 months of culture of said tree species, without cutting of the aerial parts of said pre cultured plant nurse after said pre culture, is comprised from approximately 2 to approximately 3 and the increase of the root biomass of a tree species after pre culture of only one seed of a nurse plant or only one nurse plant, and 5 months of culture of said tree species, without cutting of the aerial parts of said pre cultured plant nurse after said pre culture, is comprised from approximately 1.2 to approximately 4.

Another advantage of the present invention is that the use of only one seed or plant of a nurse plant pre cultured in a soil area wherein an exotic species or a plant having the same negative or detrimental impact on the growth of a tree species as an exotic species has been cultured, the aerial parts of said pre cultured nurse plant being uncut, is liable to significantly increase both shoot and root biomass of a cultured tree species in said soil, compared with the shoot biomass of a cultured tree species in said soil without said pre culture.

In an advantageous embodiment, the increase of the shoot biomass of a tree species after pre culture of only one seed of a nurse plant or only one nurse plant, and 5 months of culture of said tree species, with cutting of the aerial parts of said pre cultured plant nurse after said pre culture, is comprised from approximately 3 to approximately 4.

Another advantage of the present invention is that the use of only one seed or plant of a nurse plant pre cultured in a soil area wherein an exotic species or a plant having the same negative or detrimental impact on the growth of a tree species as an exotic species has been cultured, the aerial parts of said pre cultured nurse plant being cut,
is liable to significantly increase the shoot biomass of a cultured tree species in said soil, compared with:
the shoot biomass of a cultured tree species in said soil without said pre culture, and
the shoot biomass of a cultured tree species in said soil with said pre culture but without cutting said aerial parts.

In an advantageous embodiment, the increase of the root biomass of a tree species after pre culture of only one seed of a nurse plant or only one nurse plant, and 5 months of culture of said tree species, with cutting of the aerial parts of said pre cultured plant nurse after said pre culture, is comprised from approximately 1.4 to approximately 5.

Another advantage of the present invention is that the use of only one seed or plant of a nurse plant pre cultured in a soil area wherein an exotic species or a plant having the same negative or detrimental impact on the growth of a tree species as an exotic species has been cultured, the aerial parts of said pre cultured nurse plant being cut, is liable to significantly increase the root biomass of a cultured tree species in said soil, compared with:
the root biomass of a cultured tree species in said soil without said pre culture, and
the root biomass of a cultured tree species in said soil with said pre culture but without cutting said aerial parts.

In an advantageous embodiment, the increase of the shoot biomass of a tree species after pre culture of only one seed of a nurse plant or only one nurse plant, and 5 months of culture of said tree species, is comprised from approximately 3 to approximately 4 and the increase of the root biomass of a tree species after pre culture of only one seed of a nurse plant or only one nurse plant, and 5 months of culture of said tree species, with cutting of the aerial parts of said pre cultured plant nurse after said pre culture, is comprised from approximately 1.4 to approximately 5.

Another advantage of the present invention is that the use of only one seed or plant of a nurse plant pre cultured in a soil area wherein an exotic species or a plant having the same negative or detrimental impact on the growth of a tree species as an exotic species has been cultured, the aerial parts of said pre cultured nurse plant being cut,
is liable to significantly increase both the shoot and root biomass of a cultured tree species in said soil, compared with:
the shoot and root biomass of a cultured tree species in said soil without said pre culture, or the shoot and root biomass of a cultured tree species in said soil with said pre culture but without cutting said aerial parts.

The difference of the shoot and root biomass observed with the cutting of the aerial parts compared with the one obtained without cutting can be explained by a higher availability of the ectomycorrhizal colonization for the tree species when the aerial parts of the nurse plant are cut while when they are uncut, the plant nurse itself mobilizes a part of said ectomycorrhizal colonization for its own needs.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, as defined above,
wherein said nurse plant belongs to the Sarcolaenaceae family, in particular chosen among the following genus: Eremolaena,
Leptolaena, especially belonging to *Leptolaena spp*., in particular *Leptolaena bojeriana* or *Leptolaena pauciflora*,
Mediusella, Pentachlaena, Perrierodendron, Rhodolaena, Sarcolaena, Schizolaena, Xerochlamys or Xyloolaena.

The Sarcolaenaceae are a family of flowering plants endemic to Madagascar. The family includes 40 species of mostly evergreen trees and shrubs in ten genera.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, as defined above,
wherein said tree species belongs to the Phyllanthaceae family, notably to the genus Uapaca, especially belonging to *Uapaca* spp., and is in particular *Uapaca bojeri* L..

Phyllanthaceae are a family of flowering plants in the eudicot order Malpighiales and are most numerous in the tropics, with many in the south temperate zone, and in particular in Madagascar.

Other genera of the Phyllanthaceae family are the following : Actephila, Aerisilvaea, Amanoa, Andrachne, Antidesma, Aporosa, Ashtonia, Astrocasia, Baccaurea, Bischofia, Blotia, Breynia, Bridelia, Celianella, Chascotheca, Chonocentrum, Cleistanthus, Croizatia, Didymocistus, Discocarpus, Flueggea, Glochidion, Gonatogyne, Heywoodia, Hieronyma, Hymenocardia, Jablonskia, Keayodendron, Lachnostylis, Leptonema, Leptopus, Maesobotrya, Margaritaria, Meborea, Meineckia, Nothobaccaurea, Oreoporanthera, Pentabrachion, Petalodiscus, Phyllanoa, Phyllanthus, Poranthera, Protomegabaria, Pseudolachnostylis, Reverchonia, Richeria, Richeriella, Sauropus, Savia, Securinega, Spondianthus, Thecacoris, Wielandia, Zimmermannia, Zimmermanniopsis,
and also belong to the scope of the invention.

In this description the expressions "*Uapaca bojeri* L. or *Uapaca bojeri"* can be used and designate exactly the same tree species.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, as defined above,
wherein said essential ectomycorrhizal fungi to promote the growth of *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

Said ectomycorrhizal fungi have been identified and evaluated by the internal transcribed spacers sequence of the restriction fragment length polymorphism types in a soil wherein it is usually cultured such as in Madagascar, without pre culture of a nurse plant.

Said ectomycorrhizal fungi are not limitative of the ectomycorrhizal colonization found in the roots of *Uapaca bojeri* in a soil wherein it is usually cultured.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, as defined above,
wherein an exotic species has previously been cultured in said soil area, in particular *Eucalyptus camaldulensis* Dehn, said ectomycorrhizal fungi unfavorable to the growth of said *Uapaca bojeri* are ectomycorrhizal fungi of the following types: *Bondarcevomyces taxi*, *Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

In this description the expressions *"Eucalyptus camaldulensis* Dehn or *Eucalyptus camaldulensis"* can be used and designate exactly the same tree species.

In this embodiment, the soil to reforest has contained exotic species that have detrimental effects or impact on the mycorrhizal colonization of the roots of *Uapaca bojeri* giving rise to the presence of at least one ectomycorrhizal fungus unfavorable to the growth of *Uapaca bojeri* in said soil, i.e. decreasing the shoot and/or the root biomass of *Uapaca bojeri* cultured in said soil. Said at least one ectomycorrhizal fungus unfavorable to the growth of *Uapaca bojeri* in said soil is present in a relative ratio of at least 10%.

The culture of said exotic also gives rise to the decrease of at least one essential ectomycorrhizal fungus, the relative ratio of which being less than 0.1% and in particular equal to 0.

In an advantageous embodiment, at least two ectomycorrhizal fungi unfavorable to the growth of *Uapaca bojeri* in a soil wherein *Eucalyptus camaldulensis* has been cultured are present in a relative ratio of at least 10%.

In an advantageous embodiment, at least three ectomycorrhizal fungi unfavorable to the growth of *Uapaca bojeri* in a soil wherein *Eucalyptus camaldulensis* has been cultured are present in a relative ratio of at least 10%.

In an advantageous embodiment, at least two essential ectomycorrhizal fungi to the growth of *Uapaca bojeri* in a soil wherein *Eucalyptus camaldulensis* has been cultured are in a relative ratio equal or less than 0.1%.

In an advantageous embodiment, at least three essential ectomycorrhizal fungi to the growth of *Uapaca bojeri* in a soil wherein *Eucalyptus camaldulensis* has been cultured are in a relative ratio equal or less than 0.1%.

In an advantageous embodiment, at least four essential ectomycorrhizal fungi to the growth of *Uapaca bojeri* in a soil wherein *Eucalyptus camaldulensis* has been cultured are in a relative ratio equal or less than 0.1%.

In an advantageous embodiment, at least one ectomycorrhizal fungus unfavorable to the growth of *Uapaca bojeri* in a soil wherein *Eucalyptus camaldulensis* has been cultured are present in a relative ratio of at least 10% and/or at least two or three or four essential ectomycorrhizal fungi to the growth of *Uapaca bojeri* in said soil are in a relative ratio equal or less than 0.1%.

In an advantageous embodiment, at least two ectomycorrhizal fungi unfavorable to the growth of *Uapaca bojeri* in a soil wherein *Eucalyptus camaldulensis* has been cultured are present in a relative ratio of at least 10% and/or at least one or two or three or four essential ectomycorrhizal fungus (fungi) to the growth of *Uapaca bojeri* in said soil are in a relative ratio equal or less than 0.1%.

In an advantageous embodiment, at least three ectomycorrhizal fungi unfavorable to the growth of *Uapaca bojeri* in a soil wherein *Eucalyptus camaldulensis* has been cultured are present in a relative ratio of at least 10% and/or at least one or two or three or four essential ectomycorrhizal fungus (fungi) to the growth of *Uapaca bojeri* in said soil are in a relative ratio equal or less than 0.1%.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, and wherein an exotic species has previously been cultured in said soil area, in particular *Eucalyptus camaldulensis,* as defined above,
wherein said essential ectomycorrhizal fungi that are recovered in a relative ratio of about at least 10%, after said pre culture and 5 months of culture of said *Uapaca bojeri* are chosen among the following types: *Russula earlei*, *Amanita sp*., Telephoroid ectomycorrhizal sp. or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi, Russula exalbicans* or *Xerocomus chrysenteron.*

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being uncut after pre culture, and wherein an exotic species has previously been cultured in said soil area, in particular *Eucalyptus camaldulensis,* as defined above,
wherein said essential ectomycorrhizal fungi that are recovered in a relative ratio of about at least 10%, after said pre culture and 5 months of culture of said *Uapaca bojeri* are the following types: *Russula earlei*, Telephoroid ectomycorrhizal sp. and Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are the following types: *Bondarcevomyces taxi, Russula exalbicans* and *Xerocomus chrysenteron.*

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being uncut after pre culture, and wherein an exotic species has previously been cultured in said soil area, in particular *Eucalyptus camaldulensis,* said essential ectomycorrhizal fungi of the types *Russula earlei*, Telephoroid ectomycorrhizal sp. and Uncultured ectomycorrhizal homobasidiomycete clone E2 and unfavorable ectomycorrhizal fungi being of the types *Bondarcevomyces taxi, Russula exalbicans* and *Xerocomus chrysenteron,* as defined above,
wherein said *Russula earlei* is present in a relative ratio comprised from about 15 to about 25%, in particular about 23.8%, said Telephoroid ectomycorrhizal sp. is present in a relative ratio comprised from about 15 to about 25%, in particular about 20.6%, said Uncultured ectomycorrhizal homobasidiomycete clone E2 is present in a relative ratio comprised from about 10 to about 20%, in particular about 12.8%, and
said *Bondarcevomyces taxi* is present in a relative ratio comprised from about 0 to about less than 0.1%, in particular about 0%, said *Russula exalbicans* is present in a relative ratio comprised from about 0 to about less than 0.1%, in particular about 0%,
and said *Xerocomus chrysenteron* is present in a relative ratio comprised from about 0 to about less than 0.1%, in particular about 0%.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being cut after pre culture, and wherein an exotic species has previously been cultured in said soil area, in particular *Eucalyptus camaldulensis,* as defined above,
wherein said essential ectomycorrhizal fungi that are recovered in a relative ratio of about at least 10%, after said pre culture and 5 months of culture of said *Uapaca bojeri* are the following types: *Russula earlei*, *Amanita sp*., Telephoroid ectomycorrhizal sp. and Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi, Russula exalbicans* and *Xerocomus chrysenteron.*

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being cut after pre culture, and wherein an exotic species has previously been cultured in said soil area, in particular *Eucalyptus camaldulensis,* said essential ectomycorrhizal fungi being of the types *Russula earlei*, *Amanita sp*., Telephoroid ectomycorrhizal sp. and Uncultured ectomycorrhizal homobasidiomycete clone E2 and said unfavorable ectomycorrhizal fungi being of the types *Bondarcevomyces taxi, Russula exalbicans* and *Xerocomus chrysenteron* as defined above,
wherein said *Russula earlei* is present in a relative ratio comprised from about 15 to about 25%, in particular about 20.2%, said *Amanita sp.* is present in a relative ratio comprised from about 10 to about 15%, in particular about 12.1%, said Uncultured ectomycorrhizal homobasidiomycete clone E2 is present in a relative ratio comprised from about 20 to about 30%, in particular about 25.3%, said Telephoroid ectomycorrhizal sp. is present in a relative ratio comprised from about 15 to about 25%, in particular about 19.2%, and
said *Bondarcevomyces taxi* is present in a relative ratio comprised from about 0 to about less than 0.1%, in particular about 0%, said *Russula exalbicans* is present in a relative ratio comprised from about 0 to about less than 0.1%, in particular about 0%,
and said *Xerocomus chrysenteron* is present in a relative ratio comprised from about 0 to about less than 0.1%, in particular about 0%.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, as defined above, wherein an exotic species has previously been cultured in said soil area, in particular in particular *Pinus patula* Schiede ex Schtdl. & Cham, said ectomycorrhizal fungi unfavorable to the growth of said *Uapaca bojeri* are ectomycorrhizal fungi of the following types: *Bondarcevomyces taxi*, *Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

In this description the expressions *"Pinus patula* Schiede ex Schtdl. & Cham, or *Pinus patula"* can be used and designate exactly the same tree species.

In an advantageous embodiment, at least two ectomycorrhizal fungi unfavorable to the growth of *Uapaca bojeri* in a soil wherein *Pinus patula* has been cultured are present in a relative ratio of at least 10%.

In an advantageous embodiment, at least three ectomycorrhizal fungi unfavorable to the growth of *Uapaca bojeri* in a soil wherein *Eucalyptus camaldulensis* has been cultured are present in a relative ratio of at least 10%.

In an advantageous embodiment, at least two essential ectomycorrhizal fungi to the growth of *Uapaca bojeri* in a soil wherein *Eucalyptus camaldulensis* has been cultured are in a relative ratio equal or less than 0.1%.

In an advantageous embodiment, at least three essential ectomycorrhizal fungi to the growth of *Uapaca bojeri* in a soil wherein *Eucalyptus camaldulensis* has been cultured are in a relative ratio equal or less than 0.1%.

In an advantageous embodiment, at least four essential ectomycorrhizal fungi to the growth of *Uapaca bojeri* in a soil wherein *Eucalyptus camaldulensis* has been cultured are in a relative ratio equal or less than 0.1%.

In an advantageous embodiment, at least one ectomycorrhizal fungi unfavorable to the growth of *Uapaca bojeri* in a soil wherein *Eucalyptus camaldulensis* has been cultured are present in a relative ratio of at least 10% and/or at least two or three or four essential ectomycorrhizal fungus (fungi) to the growth of *Uapaca bojeri* in said soil are in a relative ratio equal or less than 0.1%.

In an advantageous embodiment, at least two ectomycorrhizal fungi unfavorable to the growth of *Uapaca bojeri* in a soil wherein *Eucalyptus camaldulensis* has been cultured are present in a relative ratio of at least 10% and/or at least one or two or three or four essential ectomycorrhizal fungus (fungi) to the growth of *Uapaca bojeri* in said soil are in a relative ratio equal or less than 0.1%.

In an advantageous embodiment, at least three ectomycorrhizal fungi unfavorable to the growth of *Uapaca bojeri* in a soil wherein *Eucalyptus camaldulensis* has been cultured are present in a relative ratio of at least 10% and/or at least one or two or three or four essential ectomycorrhizal fungi to the growth of *Uapaca bojeri* in said soil are in a relative ratio equal or less than 0.1%.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, and wherein an exotic species has previously been cultured in said soil area, in particular in particular *Pinus Patula,* as defined above,
wherein said essential ectomycorrhizal fungi that are recovered in a relative ratio of about at least 10%, after 5 months of culture of said *Uapaca bojeri* are chosen among the following types: *Russula earlei* or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi* or *Russula exalbicans.*

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being uncut after pre culture, and wherein an exotic species has previously been cultured in said soil area, in particular in particular *Pinus patula,* as defined above,
wherein said essential ectomycorrhizal fungi that are recovered in a relative ratio of about at least 10%, after said pre culture and 5 months of culture of said *Uapaca bojeri* are the following types: *Russula earlei* and Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are the following types: *Bondarcevomyces taxi* and *Russula exalbicans.*

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being uncut after pre culture, and wherein an exotic species has previously been cultured in said soil area, in particular in particular *Pinus patula,* said essential ectomycorrhizal fungi being of the types *Russula earlei* and Uncultured ectomycorrhizal homobasidiomycete clone E2 and unfavorable ectomycorrhizal fungi being of the types *Bondarcevomyces taxi* and *Russula exalbicans.* as defined above,
wherein said *Russula earlei* is present in a relative ratio comprised from about 15 to about 25%, in particular about 22.6%, said Uncultured ectomycorrhizal homobasidiomycete clone E2 is present in a relative ratio comprised from about 20 to about 30%, in particular about 28.3%, and
said *Bondarcevomyces taxi* is present in a relative ratio comprised from about 0 to about less than 0.1%, in particular about 0%, and *Russula exalbicans* is present in a relative ratio comprised from about 0 to about less than 0.1%, in particular about 0%.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being cut after pre culture, and wherein an exotic species has previously been cultured in said soil area, in particular in particular *Pinus patula,* as defined above,
wherein said essential ectomycorrhizal fungi that are recovered in a relative ratio of about at least 10%, after said pre culture and 5 months of culture of said *Uapaca bojeri* are the following types: *Russula earlei* and Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi* and *Russula exalbicans.*

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being cut after pre culture, and wherein an exotic species has previously been cultured in said soil area, in particular in particular *Pinus patula,* said essential ectomycorrhizal fungi being of the types *Russula earlei* and Uncultured ectomycorrhizal homobasidiomycete clone E2 and unfavorable ectomycorrhizal fungi being of the types *Bondarcevomyces taxi* and *Russula exalbicans.* as defined above,
wherein said *Russula earlei* is present in a relative ratio comprised from about 15 to about 25%, in particular about 17.8%, said Uncultured ectomycorrhizal homobasidiomycete clone E2 is present in a relative ratio comprised from about 30 to about 40%, in particular about 35.6 %, and
said *Bondarcevomyces taxi* is present in a relative ratio comprised from about 0 to about less than 0.1%, in particular about 0%, and *Russula exalbicans* is present in a relative ratio comprised from about 0 to about less than 0.1%, in particular about 0%.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, and wherein an exotic species has previously been cultured in said soil area, in particular *Eucalyptus camaldulensis* or *Pinus patula,* as defined above, wherein said nurse plant is devoid of a previous inoculation by an ectomycorrhizal fungus (or endomycorrhizal fungus) when pre cultured.

In this embodiment, the mycorrhizal colonization of said nurse plant observed after pre culture comes only from the interaction between the soil area and said nurse plant and:
no exogenous source of ectomycorrhizal fungi (or endomycorrhizal fungi) such as a pre or co inoculation of the seed of the nurse plant, or the nurse plant, has been carried, and
no introduction or amendment of an ectomycorrhizal fungus (or endomycorrhizal fungus) has been carried out into the soil area in an exogenous manner.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, and wherein an exotic species has previously been cultured in said soil area, in particular *Eucalyptus camaldulensis* or *Pinus patula,* as defined above,
wherein said tree species is cultured during 5 months without co-culture of a plant other than said nurse plant.

In this embodiment, in the soil area, only the tree species and the nurse plant can be present.

In an advantageous embodiment, the present invention relates to the use of at least one seed of a nurse plant or at least one nurse plant, submitted to a pre culture in a soil area, said nurse plant having been previously identified and selected, after defining and analysis of its root ectomycorrhizal compatibility with the one of said tree species in said soil area,
for promoting the growth of at least one seed or at least one plant of a tree species and modifying the ectomycorrhizal colonization of the roots of said tree species in said soil area, said pre culture lasting approximately from 3 to 6 months, in particular approximately 4 months, to give a pre cultured plant, aerial parts of said pre cultured nurse plant being optionally cut after pre culture, and wherein an exotic species has previously been cultured in said soil area, in particular *Eucalyptus camaldulensis* or *Pinus patula,* as defined above, wherein said nurse plant is devoid of a previous inoculation by an ectomycorrhizal fungus when pre cultured and said tree species is cultured during 5 months without co-culture of a plant other than said nurse plant.

In another aspect, the present invention relates to a process for promoting the growth of at least one seed or at least one plant of a tree species and modifying the ectomycorrhizal colonization of said tree species in a soil area, in particular a soil area having a volume of approximately 2,5L, comprising:
a step of identification and selection of a nurse plant among other nurses plants in said soil area after defining and analysis of its ectomycorrhizal compatibility with the one of said tree species in said soil area,
a step of determination of the ectomycorrhizal fungi of the roots of said tree species cultured in said soil area and,
a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured nurse plant being optionally cut after pre culture,
said steps of selection and of determination taking place before the pre culture of nurse plant, the ectomycorrhizal colonization C1 of the roots of said tree species cultured in said soil area, before said pre culture, being decreased by a factor comprised from about 2 to about 5 compared with the ectomycorrhizal colonization obtained in a control soil comprising said tree species, in particular due to the growth of an exotic species previously cultured in said soil area before said pre culture, said ectomycorrhizal colonization C1 comprising:
the presence of at least one ectomycorrhizal fungus, in particular of an exotic species, unfavorable to the growth of said particular tree species in said soil area in a relative ratio of at least 10%, and/or
the presence of at least one essential ectomycorrhizal fungus to promote the growth of said particular tree species in said soil area in a relative ratio equal or less than 0.1%.

The ectomycorrhizal fungi of the roots of the nurse plant that is located in the soil area to reforest are previously defined and their compatibility with those borne by the tree species is analyzed allowing selecting the nurse plant bearing at least one fungus essential to the growth of the tree species and preferably the largest number of essential fungi and more preferably the lowest number of unfavorable fungi.

Defining the ectomycorrhizal fungi of the nurse plant and the one of the tree species can be made in any order but both must be made before the selection of the nurse plant.

The identification of said ectomycorrhizal colonization and the determination of the relative content of each ectomycorrhizal fungus can be carried out with methods well known from a man skilled in the art.

In particular, said identification and determination is carried out by the internal transcribed spacers sequence of the restriction fragment length polymorphism types.

The determination step allows determining not only the ectomycorrhizal colonization C1 of the roots of said tree species but also the identification of unfavorable and essential ectomycorrhizal fungi on the root of said tree species cultured in said soil area. This can be done by the identification and the comparison of said ectomycorrhizal fungi with those found in the root of the same tree species as the one that has been cultured in a control soil, i.e. a soil that has not previously contained an exotic species or other plants having the same negative or detrimental impact on the growth of tree species as an exotic species.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, as defined above,
wherein aerial parts of said pre cultured plant are uncut after said pre culture.

In this embodiment, said tree species is introduced into the soil area to reforest after the pre culture of said nurse plant with the totality of said nurse plant. The tree species is therefore co cultured with the nurse plant after said pre culture.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, as defined above,
wherein aerial parts of said pre cultured plant are cut after said pre culture.

In this embodiment, said tree species is introduced into the soil area to reforest after the pre culture of said nurse plant with only the roots of said nurse plant, as shoot, i.e. leaves and stem have been cut. Thus said nurse plant has not been uprooted but only cut providing the advantage to leave the mycorrhizal colonization of the root nurse plant available only for the tree species and no more for the nurse plant itself.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being cut after said pre culture as defined above,
wherein said aerial parts of said cut nurse plant are used to produce essential oil or flavor, in function of the pre cultured nurse plant.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, as defined above,
wherein the ectomycorrhizal colonization of the roots of said tree species, after said pre culture of said nurse plant and 5 months of culture of said tree species, in particular a soil area having a volume of approximately 2,5L, is recovered in a range from about 70% to about 100%, said ectomycorrhizal colonization C2 comprising:
at least one unfavorable ectomycorrhizal fungi, present in said soil area before said pre culture, in a relative ratio equal or less than 0.1% and /or
the presence of at least one recovered essential ectomycorrhizal fungi in a relative ratio of about at least 10%.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, as defined above,
wherein said nurse plant belongs to the Sarcolaenaceae family, in particular chosen among the following genus: Eremolaena, Leptolaena, especially belonging to *Leptolaena spp*., in particular *Leptolaena bojeriana* or *Leptolaena pauciflora.*

Mediusella, Pentachlaena, Perrierodendron, Rhodolaena, Sarcolaena, Schizolaena, Xerochlamys or Xyloolaena.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, as defined above,
wherein said tree species belongs to the Phyllanthaceae family, notably to the genus Uapaca, especially belonging to *Uapaca* spp., and is in particular *Uapaca bojeri*

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, said tree species belonging to the Phyllanthaceae family, notably to the genus Uapaca, especially belonging to *Uapaca* spp., and is in particular *Uapaca bojeri,* as defined above,
wherein said essential ectomycorrhizal fungi to promote the growth of *Uapaca bojeri* in a control soil before pre culture of said nurse plant are ectomycorrhizal fungi of the following types: *Russula earlei*, *Amanita sp*., Telephoroid ectomycorrhizal sp. or Uncultured ectomycorrhizal homobasidiomycete clone E2.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, said tree species belonging to the Phyllanthaceae family, notably to the genus *Uapaca*, especially belonging to *Uapaca* spp., and is in particular *Uapaca bojeri,* said essential ectomycorrhizal fungi to promote the growth of *Uapaca bojeri* in a control soil before pre culture of said nurse plant being ectomycorrhizal fungi of the following types: *Russula earlei*, *Amanita sp*., Telephoroid ectomycorrhizal sp. or Uncultured ectomycorrhizal homobasidiomycete clone E2, as defined above,
wherein an exotic species has previously been cultured in said soil area, in particular *Eucalyptus camaldulensis*, said ectomycorrhizal fungi unfavorable to the growth of said *Uapaca bojeri* are ectomycorrhizal fungi of the following types: *Bondarcevomyces taxi, Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, said tree species belonging to the Phyllanthaceae family, notably to the genus *Uapaca*, especially belonging to *Uapaca* spp., and is in particular *Uapaca bojeri,* said essential ectomycorrhizal fungi to promote the growth of *Uapaca bojeri* in a control soil before pre culture of said nurse plant are ectomycorrhizal fungi of the following types: *Russula earlei*, *Amanita sp*., Telephoroid ectomycorrhizal sp. or Uncultured ectomycorrhizal homobasidiomycete clone E2, an exotic species having previously been cultured in said soil area, in particular *Eucalyptus camaldulensis,* said ectomycorrhizal fungi unfavorable to the growth of said *Uapaca bojeri* are ectomycorrhizal fungi of the following types: *Bondarcevomyces taxi, Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2, as defined above,
wherein said essential ectomycorrhizal fungi that are recovered in a relative ratio of about at least 10%, after said pre culture and 5 months of culture of said *Uapaca bojeri* are chosen among the following types: *Russula earlei*, *Amanita sp*., Telephoroid ectomycorrhizal sp. or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi, Russula exalbicans* or *Xerocomus chrysenteron.*

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, said tree species belonging to the Phyllanthaceae family, notably to the genus Uapaca, especially belonging to *Uapaca* spp., and is in particular *Uapaca bojeri,* said essential ectomycorrhizal fungi to promote the growth of *Uapaca bojeri* in a control soil before pre culture of said nurse plant being ectomycorrhizal fungi of the following types: *Russula earlei*, *Amanita sp*., Telephoroid ectomycorrhizal sp. or Uncultured ectomycorrhizal homobasidiomycete clone E2, as defined above,
wherein an exotic species has previously been cultured in said soil area, in particular *Pinus patula,* said ectomycorrhizal fungi unfavorable to the growth of said *Uapaca bojeri* are ectomycorrhizal fungi of the following types: *Bondarcevomyces taxi*, *Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

In an advantageous embodiment, the present invention relates to a process for promoting the growth of at least one seed or at least one plant of a tree species and modifying the ectomycorrhizal colonization of the roots of said tree species in said soil area, in particular a soil area having a volume of approximately 2,5L, comprising a step of identification and selection of a nurse plant among other nurses plants in said soil area after defining and analysis of its ectomycorrhizal compatibility with the one of said tree species in said soil area, a step of determination of the ectomycorrhizal fungi of the roots of said tree species cultured in said soil area and a step of pre culture of at least one seed of a said nurse plant or at least one nurse plant, in said soil area, said pre culture lasting approximately from 3 to 6 months, in particular approximately 4 months, to give a pre cultured plant, aerial parts of said pre cultured plant being optionally cut after said pre culture, said tree species belonging to the Phyllanthaceae family, notably to the genus *Uapaca*, especially belonging to *Uapaca* spp., and is in particular *Uapaca bojeri,* said essential ectomycorrhizal fungi to promote the growth of *Uapaca bojeri* in a control soil before pre culture of said nurse plant being ectomycorrhizal fungi of the following types: *Russula earlei*, *Amanita sp*., Telephoroid ectomycorrhizal sp. or Uncultured ectomycorrhizal homobasidiomycete clone E2, as defined above, an exotic species having previously been cultured in said soil area, in particular *Pinus patula,* said ectomycorrhizal fungi unfavorable to the growth of said *Uapaca bojeri* are ectomycorrhizal fungi of the following types: *Bondarcevomyces taxi*, *Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2,
wherein said essential ectomycorrhizal fungi that are recovered in a relative ratio of about at least 10%, after 5 months of culture of said *Uapaca bojeri* are chosen among the following types: *Russula earlei* or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi* or *Russula exalbicans.*

The present disclosure also describes a process defined above, wherein said nurse plant is devoid of a previous inoculation by an ectomycorrhizal fungus when pre cultured.

The present disclosure also describes a process defined above, wherein said tree species is cultured during 5 months without co-culture of a plant other than said nurse plant.

The present disclosure also describes a process defined above, comprising the following steps:
a. Identification of nurse plants present in said soil area wherein *Eucalyptus camaldulensis* or *Pinus patula* has been introduced and cultured, and analysis of the ectomycorrhizal status of said nurse plants;
b. Selecting one nurse plant, in particular *Leptolaena bojeriana* in function of its compatibility with the one of *Uapaca bojeri* to obtain a selected nurse plant;
c. Harvesting seeds of said selected nurse plant or said selected nurse plant;
d. Collecting a soil area, in particular wherein *Uapaca bojeri* has grown and eventually mixing of said soil area with a inert substrate such as sand to obtain a collected soil or a mixed soil area-inert substrate;
e. Pre culture in said collected soil or mixed soil area-inert substrate of at least one seed of selected nurse plant, in particular *Leptolaena bojeriana* or at least one selected nurse plant, in particular *Leptolaena bojeriana*; during approximately from 3 to 6 months, in particular approximately 4 months, to obtain a pre cultured nurse plant, in particular a pre cultured *Leptolaena bojeriana*;
f. Optionally after said pre culture of said selected nurse plant, cutting aerial parts of said pre cultured nurse plant, in particular of *Leptolaena bojeriana* to obtain a cut pre cultured nurse plant;
g. Introducing a seed of a tree species, in particular *Uapaca bojeri* with said optionally cut pre cultured nurse plant during 1 to 12 months, in particular 5 month, to obtain a cultured tree species, in particular a cultured *Uapaca bojeri* plant;
h. Transferring said cultured tree species, in particular a cultured *Uapaca bojeri* plant in the soil area to reforest.

The same process can be carried out without collecting the soil area in step d. and pre culture directly the nurse plant in said siol area in which a inert substrate has eventually been added. In this case, step h is not carried out.

The present disclosure also describes a process defined above, comprising the following steps:
a. Identification of nurse plants present in said soil area wherein *Eucalyptus camaldulensis* or *Pinus patula* has been introduced and cultured, and analysis of the ectomycorrhizal status of said nurse plants;
b. Selecting one nurse plant, in particular *Leptolaena bojeriana* in function of its compatibility with the one of *Uapaca bojeri* to obtain a selected nurse plant;
c. Harvesting seeds of said selected nurse plant or said selected nurse plant;
d. Collecting a soil area, in particular wherein *Uapaca bojeri* has grown and eventually mixing of said soil area with a inert substrate such as sand to obtain a collected soil or a mixed soil area-inert substrate;
e. Pre culture in said collected soil or mixed soil area-inert substrate of at least one seed of selected nurse plant, in particular *Leptolaena bojeriana* or at least one selected nurse plant, in particular *Leptolaena bojeriana*; during approximately from 3 to 6 months, in particular approximately 4 months, to obtain a pre cultured nurse plant, in particular a pre cultured *Leptolaena bojeriana*;
f. Introducing a seed of a tree species, in particular *Uapaca bojeri* with said pre cultured nurse plant during 1 to 12 months, in particular 5 month, to obtain a cultured tree species, in particular a cultured *Uapaca bojeri* plant;
g. Transferring said cultured tree species, in particular a cultured *Uapaca bojeri* plant in the soil area to reforest.

The same process can be carried out without collecting the soil area in step d. and pre culture directly the nurse plant in said siol area in which a inert substrate has eventually been added. In this case, step h is not carried out.

The present disclosure also describes a process defined above, comprising the following steps:
a. Identification of nurse plants present in said soil area wherein *Eucalyptus camaldulensis* or *Pinus patula* has been introduced and cultured, and analysis of the ectomycorrhizal status of said nurse plants;
b. Selecting one nurse plant, in particular *Leptolaena bojeriana* in function of its compatibility with the one of *Uapaca bojeri* to obtain a selected nurse plant;
c. Harvesting seeds of said selected nurse plant or said selected nurse plant;
d. Collecting a soil area, in particular wherein *Uapaca bojeri* has grown and eventually mixing of said soil area with a inert substrate such as sand to obtain a collected soil or a mixed soil area-inert substrate;
e. Pre culture in said collected soil or mixed soil area-inert substrate of at least one seed of selected nurse plant, in particular *Leptolaena bojeriana* or at least one selected nurse plant, in particular *Leptolaena bojeriana*; during approximately from 3 to 6 months, in particular approximately 4 months, to obtain a pre cultured nurse plant, in particular a pre cultured *Leptolaena bojeriana*;
f. After said pre culture of said selected nurse plant, cutting aerial parts of said pre cultured nurse plant, in particular of *Leptolaena bojeriana* to obtain a cut pre cultured nurse plant;
g. Introducing a seed of a tree species, in particular *Uapaca bojeri* with said cut pre cultured nurse plant during 1 to 12 months, in particular 5 month, to obtain a cultured tree species, in particular a cultured *Uapaca bojeri* plant;
h. Transferring said cultured tree species, in particular a cultured *Uapaca bojeri* plant in the soil area to reforest.

The same process can be carried out without collecting the soil area in step d. and pre culture directly the nurse plant in said siol area in which a inert substrate has eventually been added. In this case, step h is not carried out.

The description will be further illustrated by the following figures and examples.

### DESCRIPTION OF THE FIGURES

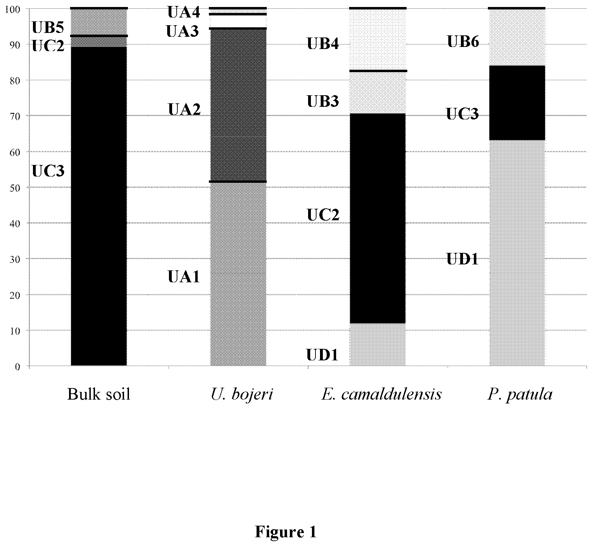

**Figure 1** presents the similarities in ectomycorrhizal communities between *U. bojeri* seedlings growing in soils collected under *Uapaca bojeri, Eucalyptus camaldulensis, Pinus patula* and from a bulk soil. Values are expressed by RFLP type percentages with regards to the soil treatments. UA1: *Russula earlei*, UD1: *Bondarcevomyces taxi,* UA2: *Amanita* sp., UA3: Telephoroid mycorrhizal sp., UC3: *Russula exalbicans,* UA4: Uncultured ECM homobasidiomycete Clone E2, UB6: *Boletellus projectellus,* UC2: *Boletus rubropunctus*, UB5: *Coltricia perennis,* UB4: *Xerocomus chrysenteron.*

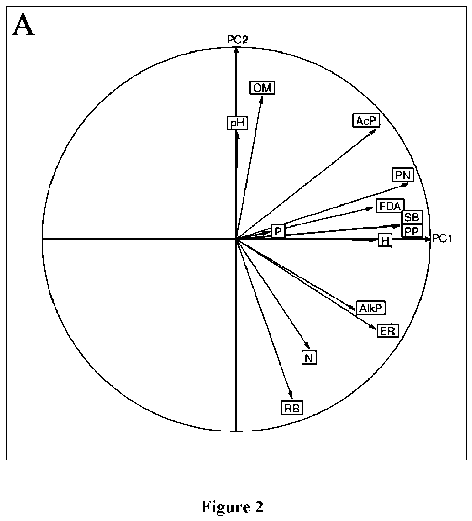

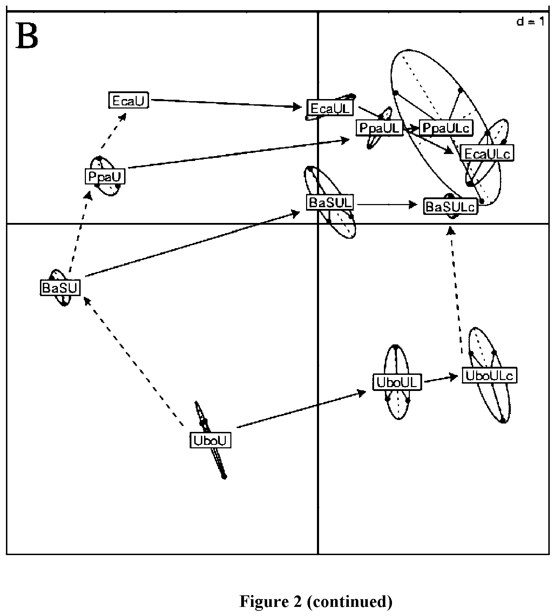

**Figure 2A** **and** **2B** present the results of the principal component analysis (PCA) on the data table of soil, plant, and microbial activity parameters.
Figure 2A: correlation circle of all the parameters. The 12 variables are : pH = pH, P = total phosphorus (mg.kg-1), N = total nitrogen (%), OM = total organic matter (%), FDA = total enzymatic activity, AcP = acid phosphatase, AlkP = alkaline phosphatase, SB = shoot biomass (g), RB = root biomass (g), ER = Ectomycorrhizal rate (%), PN = leaf nitrogen (%), PP = leaf phosphorus (mg.kg⁻¹), H = Shannon diversity index of ectomycorhizal fungi.
Figure 2B: map of sample scores on the first two principal components. Samples are coded as follows. The first three characters correspond to the soil origin: Eca = soil collected under *E. camaldulensis,* Ppa = soil collected under *P. patula,* Ubo = soil collected under *U. bojeri,* BaS = bare soil. The treatment applied to the U. bojeri seedlings is coded as folows. U *= Uapaca* plant alone, UL = *Uapaca* plant + *L. bojeriana,* Ulc = *Uapaca* plant + *L. bojeriana* cut after four months cultivation. For exemple, sample coded « EcaULc » is a *U. bojeri* seedling grown in soil collected under *E. camaldulensis* in which a plant of *L. bojeriana* was grown and cut after four month cultivation.

### EXAMPLES:

### Example 1: Study area

The field experiment was conducted within the central part of Madagascarian highland sclerophyllous forest in a forest located at 50 km to the west of Antananarivo (Arivonimamo site). The average annual rainfall was about 1398 mm with a monthly temperature varying up to 26.1°C. The vegetation is a mosaic of *U. bojeri* islands and very scattered individuals of introduced tree species, *P. patula* and *E. camaldulensis.* These trees dominate an understorey mainly composed by early-successional plant species such as *Leptolaena bojeriana*, *Leptolaena pauciflora*, *Erica* sp., *Helychrisum rusillonii, Aphloia theaformis*, *Psiadia altissima*, *Rhus taratana*, *Vaccinium emirnensis*, *Rubus apelatus* and *Trema* sp.. *L. bojeriana* was the most representative plant species in this site with a cover contribution of about 43%.

### Example 2: Analysis of the mycorrhizal status of trees and early-successional plant species

Root samples were collected during the rainy season. Root identity was ascertained by tracing from the trunk to the fine root tips. Samples of 1 to 5 g (fresh weight) of fine roots were washed under running water and stored at 4°C for further examination. Fine roots were examined for ECM infection under a dissecting microscope. Mycorrhizal features criteria following Agerer (1987 - 1996, Colour atlas of ectomycorrhizae. Schwäbisch Gmünd: Einhorn-Verlag Eduard Dietenberger) such as mantle color and structure, branching pattern and characteristics of rhizomorphs were used to categorize ectomycorrhizas into morphological type (morphotype) groups. For AM infection, fine roots were stained following the method of Phillips and Hayman (1970, Improved procedures for clearing and staining parasitic and vesicular-arbuscular mycorrhizal fungi for rapid assessment of infection. Trans Brit Mycol Soc 55: 158-160). The root pieces were placed on a slide for microscopic observation under 250 magnification (Brundrett 1991, Mycorrhizas in natural ecosystems. In: Macfayden, A., Begon, M., Fitter, A.H. (Eds.), Advances in Ecological Research, vol. 21. Academic Press Ltd., London, pp. 171-313). About fifty 1 cm root pieces were randomly chosen from each root sample collected from each plant species.

### Example 3: Bioassays of soils collected under exotic tree species (E. camaldulensis and P. patula) and the native tree species (U. bojeri)

Seven adult trees of each exotic species and of *U. bojeri* were randomly choosen in an approximately 5 ha area in the Arivonimamo forest. In order to avoid disruption of soil and more particularly changes in mycorrhizal networks, seven intact blocks of soil were collected near each adult tree (about 50 cm from the trunk). Seven additional intact blocks were collected at 10-15 m from any targeted tree species (*E. camaldulensis, P. patula* and *U. bojeri* trees) or other known ectomycorrhizal plants. Intact monoliths of soil were cut with shovel and immediately transferred into 15 cm diameter, 16 cm height plastic pots.

In addition, soil samples were taken near each soil block from the 0-10 cm layer and stored in sealed plastic bags at field moisture content at 4°C for further measurements. For each soil sample, pH of a water soil suspension was determined. The total organic carbon (TOC) was measured according to the ANNE method (Aubert 1978, Méthodes d'Analyse des sols. Edition CRDP, Marseille, p. 360) and the total nitrogen by the Kjeldhal method. The available and total phosphorus soil contents were analyzed by colorimetry (Olsen et al. 1954, Estimation of available phosphorus in soils by extraction with sodium bicarbonate. Circular, Vol 939. U.S. Depart- ment of Agriculture, Washington, DC, p. 19). Acid and alkaline phosphatase activities were measured using p-nitrophenol benzene as substrate (Schinner et al. 1996, Methods in Soil Biology. Springer-Verlag, Berlin, 426 pp. Alef, K. (1998) Estimation of the hydrolysis of fluorescein diacetate. In: Alef K. and Nannipieri P. (eds), Methods in applied soil microbiology and biochemistry. Academic Press, London, pp. 232-233), and production of the p-nitrophenol product was determined colorimetrically at 650 nm. Fluorescein diacetate (FDA) hydrolysis was assayed to provide a measurement of the microbial global activity (Remigi et al. 2008, The exotic legume tree species Acacia holosericea alters microbial soil functionalities and the structure of the Arbuscular mycorrhizal community. Appl Environ Microb 74: 1485-1493).

Seeds of *U. bojeri* collected in the Arivonimamo forest were surface sterilized in hydrogen peroxide for 10 min, rinsed and soaked in sterile distilled water for 12 h, and germinated on 1% agar. The germinating seeds were used when rootlets were 1-2 cm long. One pre-germinated seed was planted per pot filled with intact monolith of soil. The pots were randomized in the greenhouse and seedlings grown under natural light (daylight of approximately 12 h, average daily temperature of 25°C). They were watered regularly with tap water without fertilizer.

After 5 months of culturing, *U. bojeri* seedlings were gently uprooted from the pots in order to keep the root systems intact and to avoid root disruption. Then they were gently washed with running water. The percentage of ectomycorrhizal short roots (number of ectomycorrhizal short roots/total number of short roots) was assessed under a dissecting microscope by counting all single root tips. Ectomycorrhizal or non-ectomycorrhizal short roots were detected according to the presence or absence of fungal mantle and mycelium and to the presence or lack of root hairs. In each treatment, ECM root tips were classified by morphotypes based on characteristics of their mantle and extra-matrical mycelium (branching, surface colour, texture, emanating hyphae and rhizomorphs (Agerer 1995, Anatomical characteristics of identified ectomycorrhizas: an attempt towards a natural classification. In: Varma A, Hock B (eds) Mycorrhiza: structure, function, molecular biology and biotechnology. Springer, Berlin, pp 687-734). All morphological types of ectomycorrhizas were stored at -20°C in 700 µl CTAB lysis buffer (2% cetylammoniumbromide; 100 mM Tris-HCl, 20 mM EDTA, 1.4 M NaCl) before molecular analysis. Three ectomycorrhizas randomly selected from each morphotype groups were screened by RFLP analysis and one sample of each unique RFLP patterns was sequenced.

DNA was extracted from root tips using Qiagen DNeasy Plant Mini Kits (Qiagen SA, Courtaboeuf, France) following the manufacturer's recommendations. Fungal mitochondrial rDNA extracts were amplified with ML5 and ML6 primers (White et al. 1990, Amplification and direct sequencing of fungal ribosomal RNA genes for phylogenetics. In: PCR protocols: a Guide to Methods and Applications (eds Innis MA, gelfand D.H., Sninsky J.J., White T.J.), pp. 315 - 322. Academic Press Inc. San Diego, C.A) and restriction digested *Hae*III or *Hinf*I enzymes. Then one sample of each individual RFLP type was sequenced with the ABI Prism BigDye Terminator Cycle sequence kit (Applied Biosystems, Foster City, California) and analyzed on an applied Biosystems model 310 DNA sequencer (Perkin-Elmer). Sequences were aligned by using Clustal X 1.80 (Thompson et al. 1997, The ClustalX windows interface: flexible strategies for multiple sequence alignment aided by quality analysis tools. Nucl Acids Res 24: 4876- 4882) and alignment was subsequently manually corrected using Genedoc (Nicholas and Nicholas 1997, Genedoc: a toll for editing and annotating multiple sequence alignments. Distributed by the authors). All sequences were identified according to BLAST analysis at the NCBI page http://www.ncbi.nlm.nih.gov/blast/Blast.cgi, using default settings. Sequences were deposited in GenBank.

For each *U. bojeri* seedlings, the oven dry weight (1 week at 65°C) of the aerial and root part was then measured. After drying plant tissues were ground, ashed (500°C), digested in 2 ml HCL 6N and 10 ml HNO₃ N for nitrogen and then analyzed by colorimetry for P (John 1970, Colorimetric determination in soil and plant material with ascorbic acid. Soil Sci 68: 171-177). For nitrogen (Kjeldhal) determination, they were digested in 15 ml H₂SO₄ (36N) containing 50 g 1⁻¹ of salicylic acid.

### Example 4: Impact of early-successional ectomycorrhizal shrub, Leptolena bojeriana on the characteristics of soils collected under exotic tree species (E. camaldulensis and P. patula) and the native tree species (U. bojeri) and on U. bojeri early growth

Seeds of *L. bojeriana* were collected from the Arivonimamo forest. They were surface sterilized and were pre-germinated for one week in Petri-dishes on humid filter paper. A germinated seed was then transplanted into each of plastic pots filled with soil monoliths sampled as described above under exotic and native tree species. One set of pots was unplanted. There were 3 replicates for the unplanted pots and 6 for the planted pots. The pots were randomized in a greenhouse under natural light (daylight of approximately 12 h, average daily temperature of 25°C) and watered daily with deionized water. After 4 months of growth, half of the *L. bojeriana* seedlings were cut and their aerial parts discarded without any disruptions of the cultural soil and *L. bojeriana* root systems. Removal of aerial parts allowed testing the capacity of *L. bojeriana* seedlings to act as a provider of ectomycorrhizal propagules without any competitive processes between each plant species for C acquisition and consequently to reduce symbiosis costs. Then one pre-germinated seed of *U. bojeri* (treated as previously described) was planted per pot randomized in the greenhouse and seedlings were cultivated under natural light (daylight of approximately 12 h, average daily temperature of 25°C). They were watered regularly with tap water without fertilizer. There were 3 treatments: (1) control (without pre-cultivation with *L. bojeriana),* (2) pre-cultivation and dual cultivation with *L. bojeriana* (*L. bojeriana* treatment) and (3) pre-cultivation and then cultivation with *L. bojeriana* without aerial parts (*L. bojeriana* WA treatment). After 5 months of cultivation, measurements of chemical and enzymatic soil characteristics as well as *U. bojeri* ectomycorrhizal status, growth and leaf mineral contents (N, P) were determined as described before.

### Example 5: Statistical analysis

Plant growth measurements and soil characteristics were treated with one-way analysis of variance and means were compared with the Newman-Keul multiple range test (p < 0.05). The fungal colonization indexes were transformed by arcsin (√x) before statistical analysis. A principal component analysis (PCA) was applied to the soil, plant, and microbial parameters. The software used was the ade4 package (Dray and Dufour 2007) for the R software for statistical computing (R Development Core Team 2010).

### Example 6: Mycorrhizal status of trees and early-successional plant species in the Arivonimamo forest

All tree and shrub species recorded in the Arivonimamo forest formed mycorrhizas. Among these, 8 presented AM infections and 5 were found with both AM and ECM (Table 1).

**Table 1. Mycorrhizal status of trees and early-successional plant species in the Arivonimamo forest**

| **Shrub and tree species** | **Family** | **Mycorrhizal status** |
|---|---|---|
| *Leptolaena pauciflora* Baker. | Sarcolaenaceae | ECM¹ & AM² |
| *Leptolaena bojeriana* (Baill.) Cavaco. | Sarcolaenaceae | ³ECM & AM |
| *Trema* sp. | Cannabaceae | AM |
| *Vaccinium emirnense* Hook. | Ericaceae | AM |
| *Aphloia theaeformis* (Vahl.) Benn. | Aphloiaceae | AM |
| *Rhus taratana* (Baker.) H. Perrier | Anacardiaceae | AM |
| *Helychrysum rusillonii* Hochr. | Asteraceae | AM |
| *Psiadia altissima (D.C.)* Drake | Asteraceae | AM |
| *Rubus apetalus* Poir. | Rosaceae | AM |
| *Erica* sp. | Ericaceae | AM |
| *Eucalyptus camaldulensis* Dehn. | Myrtacea | ECM & AM |
| *Pinus patula* Schiede ex Schtdl. & Cham. | Pinaceae | ECM & AM |
| *Uapaca bojeri* | Euphorbiaceae | ECM & AM |

| | | |
|---|---|---|
| ¹ ECM, ectomycorrhizas; ² AM, arbuscular mycorrhizas; ³ ECM & AM, co-existence of arbuscular mycorrhizas and ectomycorrhizas. | | |

### Example 7: Impact of targeted tree species on soil chemical characteristics, ectomycorrhizal colonization and growth of U. bojeri seedlings

The highest soil acidity was recorded with the *E. camaldulensis* origin followed by *P. patula, U. bojeri* and the bulk soil (Table 2). For N and P soil contents, the opposite ranking was found with the highest values recorded with *E. camaldulensis* soil (Table 2). The total organic matter in soil was significantly higher in *U. bojeri* and the lowest value was found in the bare soil whereas *P. patula* and *E. camaldulensis* soils had intermediate TOC contents (Table 2).

The acid phosphatase and FDA activities were significantly higher in the soils collected under the targeted tree species compared to the bulk soil but these activities were higher in the soils sampled under exotic tree species than in the *U. bojeri* origins (Table 2). With the alkaline phosphatase activity, an opposite pattern was found with a higher activity in the *U. bojeri* soil followed by the *P. patula* soil and finally by the bulk and *E. camaldulensis* soils (Table 2).

**Table 2. Chemical and biochemical characteristics of rhizosphere soils collected under a native tree species (Uapaca bojeri), two exotic tree species (Pinus patula and Eucalyptus camaldulensis) and from the bare soil (control) in the Arivonimamo forest.**

| | Soil origins | | | |
|---|---|---|---|---|
| | Control | *U. bojeri* | *P. patula* | *E. camaldulensis* |
| pH (H₂O) | 5.26 (0.03) ⁽¹⁾ d ⁽²⁾ | 4.94 (0.01) c | 4.78 (0.01) b | 4.52 (0.01) a |
| Total nitrogen (%) | 0.09 (0.006) a | 0.19 (0.003) c | 0.15 (0.006) b | 0.22 (0.006) d |
| Soluble P (mg kg⁻¹) | 1.45 (0.02) a | 2.85 (0.02) c | 2.14 (0.07) b | 3.09 (0.02) d |
| Total organic matter (%) | 1.76 (0.009) a | 4.26 (0.038) d | 3.23 (0.041) b | 3.53 (0.026) c |
| Total microbial activity (µg of hydrolyzed FDA h⁻¹ g⁻¹ of soil) | 5.61 (0.05) a | 6.69 (0.25) b | 11.54 (0.65) c | 15.33 (2.05) c |
| Acid phosphatase activity (µg *p-*nitrophenol g⁻¹ of soil h⁻¹) | 130.56 (31.8) a | 314.01 (11.7) b | 867.06 (50.7) c | 586.51 (104.9) c |
| Alkaline phosphatase activity (µg *p-*nitrophenol g⁻¹ of soil h⁻¹) | 166.51 (6.91) a | 302.54 (7.44) c | 170.95 (8.47) b | 82.54 (5.59) a |

| | | | | |
|---|---|---|---|---|
| ⁽¹⁾ Standard error of the mean. ⁽²⁾ Data in the same line followed by the same letter are not significantly different according to the Newman-Keuls test (p < 0.05) | | | | |

After 5 months of culturing, shoot and root biomass, total biomass of *U. bojeri* seedlings were significantly lower in the soil collected under *E. camaldulensis* than in the other soil origins whereas the highest root and total growth were found in the *U. bojeri* soil (Table 3). Compared to the control (bulk soil), no significant effect of *P. patula* origin was recorded for the root and total biomass except for the shoot biomass (Table 3). According to the soil origins, Root / Shoot ratios ranged as follows: *U. bojeri > P. patula* > Bulk soil (control) > *E. camaldulensis* (Table 3). Nitrogen leaf contents were not significantly different among soil origins whereas phosphorus foliar content of *U. bojeri* seedlings was significant higher in the soil originating from around *U. bojeri* compared to *P. patula* soil (Table 3).

Compared to the bulk soil, the extent of the ectomycorrhizal colonization was significantly higher in the soil collected under *U. bojeri* (73.7%) and significantly lower in the *E. camaldulensis* soil (16.3%) (Table 3).

**Table 3. Response of U. bojeri seedling growth and ectomycorrhizal colonization in soils from different tree species (Uapaca bojeri, Pinus patula and Eucalyptus camaldulensis) and from the bare soil (control) after 5 months culturing in glasshouse conditions.**

| | Soil origins | | | |
|---|---|---|---|---|
| | Control | *U. bojeri* | *P. patula* | *E. camaldulensis* |
| Shoot biomass (mg dry weight) | 131 (11) ⁽¹⁾ b ⁽²⁾ | 125 (15) b | 85 (12) a | 83 (9) a |
| Root biomass (mg dry weight) | 113 (12) b | 295 (35) c | 119 (10) b | 27 (4) a |
| Total biomass (mg dry weight) | 244 (12) b | 419 (48) c | 205 (22) b | 110 (8) a |
| Root: Shoot ratio | 0.88 (0.15) b | 2.37 (0.16) d | 1.42 (0.12) c | 0.34 (0.08) a |
| N leaf mineral content (mg per plant) | 0.89 (0.06) a | 0.85 (0.1) a | 0.65 (0.09) a | 0.65 (0.07) a |
| P leaf mineral content (mg per plant) | 71.1 (7.3) ab | 94.1 (9.9) b | 58.9 (8.7) a | 62.3 (7.3) ab |
| Ectomycorrhizal colonization (%) | 36.1 (2.08) b | 73.7 (3.18) c | 29.3 (5.55) ab | 16.3 (2.40) a |

| | | | | |
|---|---|---|---|---|
| ⁽¹⁾ Standard error of the mean. ⁽²⁾ Data in the same line followed by the same letter are not significantly different according to the Newman-Keuls test (p < 0.05). | | | | |

Structures of ectomycorrhizal communities associated with *U. bojeri* root systems in the different soil origins were significantly different (Table 4, Fig. 1). The RFLP types UA1 (*Russula earlei*), UA2 *(Amanita* sp.), UA3 (Thelephoroid symbiont) and UA4 (uncultured ECM fungus) were only recorded on *U. bojeri* seedlings grown in *U. bojeri* soil whereas, in the soils collected under exotic tree species, UD1 (*Bondarcevomyces*), UC3 (*Russula exalbicans*) and UB6 (*Boletellus projectellus*) were found. In the bare soil, the RFLP type UC3 was mainly detected and two other types, UC2 (*Boletus rubropunctus*) and UB5 (*Coltricia perennis*) at lower abundances (Fig. 1). The RFLP type UB4 (*Xerocomus chrysenteron*) was only recorded in the *E. camaldulensis* soil treatment (Fig. 1).

**Table 4. Identification by ITS sequence of RFLP types for ectomycorrhizas collected on U. bojeri seedling after 5 month culturing in glasshouse conditions on soils collected under a native tree species (Uapaca bojeri), two exotic tree species (Pinus patula and Eucalyptus camaldulensis) and from the bare soil (control) in the Arivonimamo forest**

| RFLP types | GenBank accession number | Closest GenBank species | BLAST expected value |
|---|---|---|---|
| UA1 | AF518722 | *Russula earlei* | 2e-144 |
| UD1 | DQ534583 | *Bondarcevomyces taxi* | 3e-138 |
| UA2 | AM117659 | *Amanita* sp. | 0.0 |
| UA3 | AJ509798 | Telephoroid mycorrhizal sp. | 1e-154 |
| UC3 | AY293269 | *Russula exalbicans* | 2e-170 |
| | | Uncultured ECM | |
| UA4 | AY157720 | homobasidiomycete Clone E2 | 0.0 |
| UB6 | DQ534582 | *Boletellus projectellus* | 0.0 |
| UC2 | FJ480421 | *Boletus rubropunctus* | 2e-171 |
| UB5 | none | *Coltricia perennis* | 2e-141 |
| UB4 | AD001659 | *Xerocomus chrysenteron* | 4e-173 |

### Example 7: Responses of soil characteristics and U. bojeri growth to the L. bojeriana cultivation

A data table with 36 rows and 12 columns was constructed with the soil, plant, and microbial activity parameters. The 12 variables were: pH, soluble phosphorus, total nitrogen and total organic matter, total microbial activity, acid and alkaline phosphatase activities, shoot and root biomass of *U. bojeri* seedlings, ectomycorrhizal rate, leaf nitrogen and phosphorus contents, and the Shannon diversity index of the ectomycorhizal fungal morphotypes. The 36 rows corresponded to three samples of the four soil origins: soil collected under *E. camaldulensis*, *P. patula, U. bojeri,* or bare soil. For each soil origin, three treatments were considered: *U. bojeri* seedling was planted alone, with a *L. bojeriana* seedling, or with a *L. bojeriana* seedling that aerial part was cut after four months of cultivation, but keeping intact its root system. The resulting data table was submitted to a principal component analysis (PCA) to describe the main structures of this dataset.

The Figure 2 showed the results of this PCA. The upper part (Fig. 2A) graphic was the correlation circle of all the parameters, and the lower part graphic (Fig. 2B) was the map of sample scores on the first two principal components. The correlation circle (Fig. 2A) showed that the first principal component (PC1) was well correlated to plant growth, with better growth toward the right of the graphic (shoot biomass, leaf phosphorus and leaf nitrogen contents) and also to the microbial activities (total microbial activity, acid and alkaline phosphatase activity), to the ectomycorrhizal rate, and to the Shannon diversity index of ectomycorhizal fungi. The second principal component (PC2) was negatively correlated to root biomass increase and soil total nitrogen (downward arrows) and positively to organic matter and pH (upward arrows).

The map of sample scores (Fig. 2B) showed on the first principal component (PC1) the very strong effect of the *L. bojeriana* plant (solid arrows pointing right). This effect was positive, as it corresponded to an increase of *U. bojeri* seedling growth, of microbial activities, and of ectomycorhizal fungal diversity. This effect was highest when the *Leptolena* plant was cut and only the root system was left before planting *U. bojeri* seedlings. It was also interesting to notice that this effect was the same for bare soil, for soils collected under exotic tree species or for soil collected under a *Uapaca* adult tree. On the same graphic (Fig. 2B), the second principal component (PC2) showed the soil origin effect (dotted arrows pointing upward), corresponding to the negative influence of exotic tree species (*E. camaldulensis, P. patula*) on root biomass. Root biomass was higher in soils collected under *U. bojeri* adult tree, and lower in soils collected under exotic tree species. Bare soils have an intermediate position. Conversely, pH and total organic matter are higher in soils collected under exotic tree species.

For each soil origins, the impact of *L. bojeriana* (with or without aerial parts) on soil characteristics, *U. bojeri* growth and ectomycorrhizal communities was indicated in tables 5, 6 and 7. For the bulk soil origin and compared to the control, the treatment with *L. bojeriana* without aerial parts provided the highest positive effects on pH, soluble P, soil N content, organic matter content and on microbial enzymatic activities (Table 5).

**Table 5. Effect of L. bojeriana / U. bojeri succession (pre-cultivation with L. bojeriana and dual-cultivation with L. bojeriana seedlings with aerial parts or without aerial parts) on soil chemical characteristics and enzymatic activities.**

| Treatments | pH H₂O | Sol P ⁽⁴⁾ | Total N ⁽⁵⁾ | Total OM ⁽⁶⁾ | FDA ⁽⁷⁾ | Ac P ⁽⁸⁾ | Alk P ⁽⁹⁾ |
|---|---|---|---|---|---|---|---|
| **Bulk soil** | | | | | | | |
| Control ⁽¹⁾ | 5.7 ⁽¹⁰⁾ | 2.00 | 0.022 | 4.20 | 32.0 | 498.8 | 274.6 |
| | (0.01) a ⁽¹¹⁾ | (0.06) a | (0.001) a | (0.06) a | (6.4) a | (31.9) a | (6.2) a |
| *L. bojeriana* ⁽²⁾ | 5.9 | 4.47 | 0.024 | 6.33 | 46.9 | 1046.4 | 359.5 |
| | (0.01) b | (0.09) b | (0.001) a | (0.04) b | (1.4) ab | (52.1) b | (113.7) ab |
| *L. bojeriana* | 6.2 | 5.50 | 0.103 | 9.65 | 57.5 | 1334.5 | 383.7 |
| WA ⁽³⁾ | (0.02) c | (0.11) c | (0.001) b | (0.03) c | (3.6) b | (82.6) c | (22.1) b |
| ***U. bojeri* soil** | | | | | | | |
| Control | 5.4 | 5.35 | 0.301 | 7.32 | 5.2 | 715.6 | 404.1 |
| | (0.01) b | (0.03) a | (0.001) a | (0.01) a | (0.36) a | (19.5) a | (11.6) a |
| *L. bojeriana* | 5.4 | 6.80 | 0.412 | 8.32 | 49.8 | 980.7 | 512.2 |
| | (0.01) b | (0.06) c | (0.001) b | (0.04) b | (3.4) b | (23.4) b | (22.3) b |
| *L. bojeriana* | 5.3 | 6.32 | 0.423 | 8.72 | 63.5 | 1044.3 | 582.5 |
| WA | (0.02) a | (0.06) b | (0.001) c | (0.07) c | (2.2) c | (24.9) b | (17.7) b |
| ***E.*** | | | | | | | |
| ***camaldulensis*** | | | | | | | |
| **soil** | | | | | | | |
| Control ⁽¹⁾ | 5.3 | 9.23 | 0.054 | 15.76 | 6.1 | 1213.5 | 214.3 (5.6) |
| | (0.007) a | (0.03) c | (0.001) a | (0.09) b | (1.6) a | (19.9) a | a |
| *L. bojeriana* ⁽²⁾ | 6.3 | 4.43 | 0.064 | 15.80 | 21.4 | 1447.2 | 417.1 |
| | (0.009) c | (0.09) b | (0.002) b | (0.06) b | (3.1) b | (48.1) b | (26.3) b |
| *L. bojeriana* | 5.4 | 3.70 | 0.071 | 14.25 | 68.3 | 1597.6 | 394.4 |
| WA ⁽³⁾ | (0.006) b | (0.06) a | (0.001) c | (0.03) a | (5.3) c | (8.3) c | (43.6) b |
| ***P. patula* soil** | | | | | | | |
| Control | 6.2 | 3.36 | 0.087 | 14.47 | 22.2 | 558.3 | 288.5 (4.5) |
| | (0.01) a | (0.09) a | (0.001) b | (0.09) b | (3.8) a | (55.2) a | a |
| *L. bojeriana* | 6.3 | 7.60 | 0.073 | 14.05 | 100.4 | 1257.1 | 567.9 |
| | (0.01) a | (0.11) c | (0.001) a | (0.03) a | (8.6) c | (37.5) b | (18.3) c |
| *L. bojeriana* | 6.2 | 4.40 | 0.090 | 14.68 | 66.4 | 1594.9 | 331.4 |
| WA | (0.01) a | (0.06) b | (0.001) b | (0.06) b | (4.4) b | (49.3) c | (14.5) b |

| | | | | | | | |
|---|---|---|---|---|---|---|---|
| ⁽¹⁾ *U. bojeri* without pre- and dual cultivation with *L. bojeriana.* ^{(*2*)} Pre-cultivation with *L. bojeriana* and dual-cultivation with *L. bojeriana* seedlings with aerial parts. ⁽³⁾ pre-cultivation with *L. bojeriana* and dual-cultivation with *L. bojeriana* seedlings without aerial parts (WA). ⁽⁴⁾ Soluble Phosphorus (mg kg⁻¹). ⁽⁵⁾ Total Nitrogen (%). ⁽⁶⁾ Total Organic Matter (%). ⁽⁷⁾ Total microbial activity (µg of hydrolyzed FDA h⁻¹ g⁻¹ of soil). ⁽⁸⁾ Acid phosphatase activity (µg *p-*nitrophenol g⁻¹ of soil h⁻¹). ⁽⁹⁾ Alkaline phosphatase activity (µg *p*-nitrophenol g⁻¹ of soil h⁻¹). ⁽¹⁰⁾ Standard error of the mean. ⁽¹¹⁾ Data in the same column and for each soil origin followed by the same letter are not significantly different according to the Newman-Keuls test (p < 0.05). | | | | | | | |

The dual cultivation of *L. bojeriana* with or without aerial parts significantly improved shoot and root biomass and mineral nutrition of *U. bojeri* seedlings (N, P) (Table 6).

Ectomycorrhizal colonization was significantly improved when the dual cultivation was performed with *L. bojeriana* without aerial parts (Table 6).

**Table 6. Effect of L. bojeriana / U. bojeri succession (pre-cultivation with L. bojeriana and dual-cultivation with L. bojeriana seedlings with aerial parts or without aerial parts) on the growth and ectomycorrhizal colonization of U. bojeri seedlings in soils collected under Uapaca bojeri, Eucalyptus camaldulensis, Pinus patula and from a bulk soil after 5 month culture in glasshouse conditions**

| Treatments | SB ⁽⁴⁾ | RB ⁽⁵⁾ | RB:SB (6) | N ⁽⁷⁾ | P ⁽⁸⁾ | ECM ⁽⁹⁾ |
|---|---|---|---|---|---|---|
| **Bulk soil** | | | | | | |
| Control ⁽¹⁾ | 131 | 113 | 0.88 | 0.89 | 71.1 | 36 |
| | (11) ⁽¹⁰⁾ a ^{( 11)} | (12) a | (0.13) b | (0.06) a | (7.3) a | (2.1) a |
| *L. bojeriana* ⁽²⁾ | 277 | 140 | 0.51 | 3.02 | 253.4 | 42 |
| | (11) b | (10) ab | (0.04) a | (0.12) b | (10.9) b | (6) a |
| *L. bojeriana* WA ⁽³⁾ | 309 | 166 | 0.55 | 3.08 | 332.1 | 90.3 |
| | (26) b | (3) b | (0.04) ab | (0.27) b | (29.1) b | (3.2) b |
| ***U. bojeri* soil** | | | | | | |
| Control | 125 | 295 | 2.37 | 0.85 | 94.1 | 73.7 |
| | (15) a | (35) a | (0.16) b | (0.1) a | (9.9) a | (3.2) a |
| *L. bojeriana* | 222 | 242 | 1.21 | 2.14 | 197.7 | 78 |
| | (38) ab | (38) a | (0.33) a | (0.32) b | (34.1) b | (2.1) a |
| *L. bojeriana* WA | 332 | 219 | 0.67 | 3.58 | 303.9 | 90.7 |
| | (19) b | (39) a | (0.14) a | (0.19) c | (14.1) c | (2.4) b |
| ***E. camaldulensis* soil** | | | | | | |
| Control ⁽¹⁾ | 83 | 27 | 0.34 | 0.65 | 62.3 | 16.3 |
| | (0.9) a | (4) a | (0.08) a | (0.07) a | (7.3) a | (2.4) a |
| *L. bojeriana* ⁽²⁾ | 233 | 99 | 0.45 | 2.30 | 194.6 | 65.3 |
| | (41) b | (6) b | (0.09) a | (0.41) b | (35.5) b | (3.3) b |
| *L. bojeriana* WA ⁽³⁾ | 250 | 129 | 0.57 | 3.17 | 268.6 | 79.3 |
| | (42) b | (12) b | (0.17) a | (0.57) b | (44.9) b | (4.1) b |
| *P. patula* soil | | | | | | |
| Control | 85 | 119 | 1.42 | 0.65 | 58.9 | 29.3 |
| | (12) a | (10) a | (0.12) b | (0.09) a | (8.7) a | (5.5) a |
| *L. bojeriana* | 233 | 146 | 0.62 | 2.28 | 181.3 | 30.3 |
| | (9) b | (27) a | (0.11) a | (0.10) b | (5.7) b | (2.4) a |
| *L. bojeriana* WA | 333 | 127 | 0.41 | 3.90 | 278.1 | 65.3 |
| | (66) b | (7) a | (0.08) a | (0.78) b | (53.9) b | (1.5) b |

| | | | | | | |
|---|---|---|---|---|---|---|
| ⁽¹⁾ *U. bojeri* without pre- and dual cultivation with *L. bojeriana.* ⁽²⁾ Pre-cultivation with *L. bojeriana* and dual-cultivation with *L. bojeriana* seedlings with aerial parts. ⁽³⁾ Pre-cultivation with *L. bojeriana* and dual-cultivation with *L. bojeriana* seedlings without aerial parts. ⁽⁴⁾ Shoot biomass (mg dry weight). ⁽⁵⁾ Root biomass (mg dry weight). ⁽⁶⁾ Root: Shoot ratio. ⁽⁷⁾ N leaf mineral content (mg per plant). ⁽⁸⁾ P leaf mineral content (mg per plant). ⁽⁹⁾ Ectomycorrhizal colonization (%). ⁽¹⁰⁾ Standard error of the mean. ⁽¹¹⁾ Data in the same column and for each soil origin followed by the same letter are not significantly different according to the Newman-Keuls test (p < 0.05). | | | | | | |

Strong modifications in the composition of ectomycorrhizal communities occurred in the treatments with *L. bojeriana* (Table 7). RFLP types, UC3, UC2 and UB5 recorded in the control treatment, were not found in the dual cultivation treatments and replaced by the RFLP types UA1, UA2 and UB4. The RFLP type UB6 was only recorded in the treatment with entire *L. bojeriana* seedlings (Table 7).

**Table 7. Relative abundance of RFLP types harvested in U. bojeri seedlings in the cultural patterns with L. bojeriana (pre-cultivation with L. bojeriana and dual-cultivation with L. bojeriana seedlings with aerial parts or without aerial parts) in soils collected under Uapaca bojeri, Eucalyptus camaldulensis, Pinus patula and from a bulk soil after 5 month culture in glasshouse conditions**

| Treatments | Relative abundance of RFLP types (%) | | | | | | | | | |
|---|---|---|---|---|---|---|---|---|---|---|
| | UA1 | UD1 | UA2 | UA3 | UC3 | UA4 | UB6 | UC2 | UB5 | UB4 |
| **Bulk soil** | | | | | | | | | | |
| Control ⁽¹⁾ | 0.0 | 0.0 | 0.0 | 0.0 | 89.4 | 0.0 | 0.0 | 3.1 | 7.5 | 0.0 |
| *L. bojeriana* ⁽²⁾ | 26.5 | 0.0 | 27.9 | 0.0 | 0.0 | 0.0 | 7.4 | 0.0 | 0.0 | 38.2 |
| *L. bojeriana* WA ⁽³⁾ | 19.3 | 0.0 | 49.5 | 0.0 | 0.0 | 0.0 | 0.0 | 0.0 | 0.0 | 31.2 |
| ***U. bojeri* soil** | | | | | | | | | | |
| Control | 51.5 | 0.0 | 43.0 | 3.7 | 0.0 | 1.8 | 0.0 | 0.0 | 0.0 | 0.0 |
| *L. bojeriana* | 13.0 | 0.0 | 18.5 | 0.0 | 19.6 | 19.6 | 29.3 | 0.0 | 0.0 | 0.0 |
| *L. bojeriana* WA | 14.7 | 0.0 | 16.7 | 0.0 | 11.8 | 22.5 | 34.3 | 0.0 | 0.0 | 0.0 |
| ***E. camaldulensis* soil** | | | | | | | | | | |
| Control ⁽¹⁾ | 0.0 | 11.9 | 0.0 | 0.0 | 58.7 | 0.0 | 11.9 | 0.0 | 0.0 | 17.5 |
| *L. bojeriana* ⁽²⁾ | 23.8 | 0.0 | 0.0 | 20.6 | 0.0 | 12.8 | 42.8 | 0.0 | 0.0 | 0.0 |
| *L. bojeriana* WA ⁽³⁾ | 20.2 | 0.0 | 12.1 | 19.2 | 0.0 | 25.3 | 23.2 | 0.0 | 0.0 | 0.0 |
| ***P. patula* soil** | | | | | | | | | | |
| Control | 0.0 | 63.2 | 0.0 | 0.0 | 20.8 | 0.0 | 16.0 | 0.0 | 0.0 | 0.0 |
| *L. bojeriana* | 22.6 | 0.0 | 0.0 | 0.0 | 0.0 | 28.3 | 49.1 | 0.0 | 0.0 | 0.0 |
| *L. bojeriana* WA | 17.8 | 0.0 | 0.0 | 0.0 | 0.0 | 35.6 | 46.6 | 0.0 | 0.0 | 0.0 |

| | | | | | | | | | | |
|---|---|---|---|---|---|---|---|---|---|---|
| ⁽¹⁾ *U. bojeri* without pre- and dual cultivation with *L. bojeriana.* ⁽²⁾ Pre-cultivation with *L. bojeriana* and dual-cultivation with *L. bojeriana* seedlings with aerial parts. ⁽³⁾ pre-cultivation with *L. bojeriana* and dual-cultivation with *L. bojeriana* seedlings without aerial parts. UA1: *Russula earlei*, UD1: *Bondarcevomyces taxi*, UA2: *Amanita* sp., UA3: Telephoroid mycorrhizal sp., UC3: *Russula exalbicans,* UA4: Uncultured ECM homobasidiomycete Clone E2, UB6: *Boletellus projectellus,* UC2: *Boletus rubropunctus,* UB5: *Coltricia perennis,* UB4: *Xerocomus* | | | | | | | | | | |

For the *U. bojeri* soil origin, dual cultivation with entire *L. bojeriana* seedlings increased all the measured soil parameters except for pH (Table 5). Eliminating the aerial parts of *L. bojeriana* seedlings led to higher increases of N, organic matter soil contents and FDA activity but to a lower enhancement of soil soluble P content (Table 5). Dual cultivation had significantly improved plant nutrient (N and P) uptake with highest data for the treatment without aerial parts (Table 6). No significant effect has been found on root growth and root / shoot ratio but shoot growth of *U. bojeri* seedlings was significantly improved with *L. bojeriana* without aerial parts. Ectomycorrhizal colonization was significantly increased when *U. bojeri* seedlings were cultivated with *L. bojeriana* without aerial parts (Table 6). This positive impact was also recorded on the composition of ectomycorrhizal communities with the same RFLP types (except for UA3) as those found in the control treatment (UA1, UA2 and UA4) and two others only detected with the presence of *L. bojeriana* seedlings (Table 7). With *E. camaldulensis* soil, dual cultivation treatments significantly improved soil pH, nitrogen content and enzymatic activities with highest effects found in *L. bojeriana* seedlings without aerial parts for soil nitrogen content and FDA activity (Table 5). Opposite effects have been found for soil P content and soil organic matter (depressive effect provided by *L. bojeriana* seedlings without aerial parts). Dual cultivation treatments have enhanced the growth of *U. bojeri* seedlings and ectomycorrhizal colonization but no significant differences have been found between both *L. bojeriana* treatments (with or without aerial parts) and no effects have been recorded on the root / shoot values (Table 6). The presence of *L. bojeriana* seedlings allowed the development of some RFLP types not detected in the control treatment (UA1, UA2, UA3, UA4), increased the establishment of UB6 but limited UB4 multiplication (Table 7).

For the *P. patula* soil origin, dual cultivation treatments significantly improved soil P content and enzymatic activities whereas the presence of entire *L. bojeriana* seedlings significantly decreased soil nitrogen and organic matter contents (Table 5). *U. bojeri* shoot growth and leaf foliar contents (N, P) have been significantly promoted by *L. bojeriana* seedlings (entire or not) (Table 6) and ectomycorrhizal colonization was higher in the dual cultivation treatment involving *L. bojeriana* seedlings without aerial parts (Table 6). Only UB6 RFLP type was detected in all the treatments whereas UC3 recorded in the control treatment was absent in the dual cultivation treatments (Table 7). An opposite pattern was found with UA1 and UA4 RFLP types (Table 7).

These results clearly show that (i) the introduction of exotic tree species induces significant changes in the soil chemical characteristics, microbial activities and on ectomycorrhizal communities, (ii) exotic-invaded soil significantly reduces the early growth and ectomycorrhization of *U. bojeri* seedlings and (iii) ectotrophic early successionnal shrub species such as *L. bojeriana* could lower these negative effects provided by an exotic species such as *E. camaldulensis* and *P. patula* by facilitating ectomycorrhizal establishment and, consequently improved the *U. bojeri* early growth.

## Claims

1. Use of at least one seed of a nurse plant in a soil area or of at least one nurse plant in a soil area, wherein an exotic species has previously been cultured, as a preculture, for promoting the growth of at least one seed or of at least one plant of a tree species in said soil area, by modifying the ectomycorrhizal colonization of the roots of said tree species in said soil area,
wherein
(a) said preculture is lasting from 3 to 6 months in said soil area, in particular 4 months, to give a pre cultured nurse plant, aerial parts of said pre cultured nurse plant being cut after pre culture,
(b) said at least one seed or at least one plant of said tree species is introduced into said soil area after said pre culture,
said tree species having an ectomycorrhizal colonization of the roots before pre culture, in said soil area,(C1), C1 being decreased by a factor comprised from 2 to 5 as compared with ectomycorrhizal colonization of the roots of said tree species obtained in a control soil comprising said tree species, due to the growth of said exotic species in said soil area before said pre culture,
C1 comprising 0.1% or less than 0.1% of at least one ectomycorrhizal fungus essential for promoting the growth of said tree species in said soil,
the ectomycorrhizal colonization of the roots of said tree species after said pre culture of said nurse plant and 5 months of culture of said tree species in said soil area, (C2), being recovered in a range from 70% to 100%,
C2 comprising:
at least one unfavorable ectomycorrhizal fungus, present in said soil area before said pre culture, in a relative ratio equal or less than 0.1%
and/or at least one recovered essential ectomycorrhizal fungus in a relative ratio of at least 10%,
wherein said nurse plant is *Leptolaena Bojeriana,* said tree species is *Uapaca bojeri L.*, and said exotic species is *Pinus patula* or *Eucalyptus calmadulensis*

2. Use according to claim 1, wherein only one seed of a nurse plant or only one nurse plant is pre cultured.

3. Use according to claim 1 or 2, wherein said essential ectomycorrhizal fungi to promote the growth of *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

4. Use according to claim 3 , wherein an exotic species *Eucalyptus calmadulensis* Dehn has previously been cultured in said soil area, , said ectomycorrhizal fungi unfavorable to the growth of said *Uapaca bojeri* are ectomycorrhizal fungi of the following types: *Bondarcevomyces taxi*, *Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

5. Use according to claim 4 , wherein said essential ectomycorrhizal fungi that are recovered in a relative ratio of at least 10%, after said pre culture and 5 months of culture of said *Uapaca bojeri* are chosen among the following types: *Russula earlei*, *Amanita sp*., Telephoroid ectomycorrhizal sp. or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi, Russula exalbicans* or *Xerocomus chrysenteron.*

6. Use according to claim 3 , wherein an exotic species *Pinus patula* Schiede ex Schtdl. & Cham has previously been cultured in said soil area, , said ectomycorrhizal fungi unfavorable to the growth of said *Uapaca bojeri* are ectomycorrhizal fungi of the following types: *Bondarcevomyces taxi, Russula exalbicans* or *Xerocomus chrysenteron* and said essential ectomycorrhizal fungi to promote the growth of said *Uapaca bojeri* are ectomycorrhizal fungi of the following types: *Russula earlei*, *Amanita sp*., Telephoroid ectomycorrhizal sp. or Uncultured ectomycorrhizal homobasidiomycete clone E2.

7. Use according to claim 6 , wherein said essential ectomycorrhizal fungi that are recovered in a relative ratio of about at least 10%, after 5 months of culture of said *Uapaca bojeri* are chosen among the following types: *Russula earlei* or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi* or *Russula exalbicans.*

8. Process for promoting the growth of at least one seed or at least one plant of a tree species in a soil area, wherein an exotic species has previously been cultured, and modifying the ectomycorrhizal colonization of the roots of said tree species in said soil area, in particular a soil area having a volume of approximately 2,5L, comprising:
(1) a step of pre culture of at least one seed of a nurse plant in a said soil area or of at least one nurse plant, in said soil area, said pre culture lasting from 3 to 6 months, in particular 4 months, to give a pre cultured plant, aerial parts of said pre cultured nurse plant being cut after pre culture,
(2) a step of introducing said at least one seed or said at least one plant of a tree species into said soil area,
the ectomycorrhizal colonization of the roots of said tree species cultured in said soil area, before or without said pre culture (C1), being decreased by a factor comprised from 2 to 5, as compared with the ectomycorrhizal colonization obtained in a control soil comprising said tree species, due to the growth of an exotic species in said soil area before said pre culture, said ectomycorrhizal colonization (C1) comprising:
at least one essential ectomycorrhizal fungus to promote the growth of said tree species in said soil area in a relative ratio equal or less than 0.1%;
the ectomycorrhizal colonization of the roots of said tree species, after said pre culture of said nurse plant and 5 months of culture of said tree species (C2), in particular in a soil area having a volume of approximately 2,5L, being recovered in a range from about 70% to about 100%, said ectomycorrhizal colonization (C2) comprising:
in a relative ratio, equal or less than 0.1% of said at least one unfavorable ectomycorrhizal fungi, present in said soil area before said pre culture, and/or in a relative ratio, of at least 10%, of at least one recovered of said essential ectomycorrhizal fungi.
wherein said nurse plant is *Leptolaena Bojeriana,* said tree species is *Uapaca bojeri L.,* and said exotic species is *Pinus patula* or *Eucalyptus calmadulensis.*

9. Process according to claim 8, wherein said essential ectomycorrhizal fungi to promote the growth of *Uapaca bojeri* in a control soil before pre culture of said nurse plant are ectomycorrhizal fungi of the following types: *Russula earlei, Amanita sp.,* Telephoroid ectomycorrhizal sp. or Uncultured ectomycorrhizal homobasidiomycete clone E2.

10. Process according to claim 9, wherein an exotic species has previously been cultured in said soil area, in particular *Eucaliptus calmadulensis,* said ectomycorrhizal fungi unfavorable to the growth of said *Uapaca bojeri* are ectomycorrhizal fungi of the following types: *Bondarcevomyces taxi, Russula exalbicans* or *Xerocomus chrysenteron* and said essential ectomycorrhizal fungi to promote the growth of said *Uapaca bojeri* are ectomycorrhizal fungi of the following types: *Russula earlei, Amanita sp.,* Telephoroid ectomycorrhizal sp. or Uncultured ectomycorrhizal homobasidiomycete clone E2.

11. Process according to claim 10, wherein said essential ectomycorrhizal fungi that are recovered in a relative ratio of at least 10%, after said pre culture and 5 months of culture of said *Uapaca bojeri* are chosen among the following types: *Russula earlei, Amanita sp.,* Telephoroid ectomycorrhizal sp. or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi, Russula exalbicans* or *Xerocomus chrysenteron.*

12. Process according to claim 9, wherein an exotic species has previously been cultured in said soil area, in particular *Pinus Patula,* said ectomycorrhizal fungi unfavorable to the growth of said *Uapaca bojeri* are ectomycorrhizal fungi of the following types: *Bondarcevomyces taxi, Russula exalbicans* or *Xerocomus chrysenteron* and said essential ectomycorrhizal fungi to promote the growth of said *Uapaca bojeri* are ectomycorrhizal fungi of the following types: *Russula earlei, Amanita sp.,* Telephoroid ectomycorrhizal sp. or Uncultured ectomycorrhizal homobasidiomycete clone E2.

13. Process according to claim 12, wherein said essential ectomycorrhizal fungi that are recovered in a relative ratio of at least 10%, after 5 months of culture of said *Uapaca bojeri* are chosen among the following types: *Russula earlei* or Uncultured ectomycorrhizal homobasidiomycete clone E2 and unfavorable ectomycorrhizal fungi in a relative ratio equal or less than 0.1% after said pre culture and 5 months of culture of said *Uapaca bojeri* are chosen among the following types: *Bondarcevomyces taxi* or *Russula exalbicans.*

## Patentansprüche

1. Verwendung von mindestens einem Samen einer Ammenpflanze in einem Bodenbereich oder von mindestens einer Ammenpflanze in einem Bodenbereich, in dem eine exotische Spezies vorher als Vorkultur gezüchtet worden ist, zum Unterstützen des Wachstums von mindestens einem Samen oder von mindestens einer Pflanze einer Baumspezies in einem Bodenbereich durch Modifizieren der Ektomykorrhizakolonisierung der Wurzeln der Baumspezies in dem Bodenbereich,
wobei
(a) wobei die Vorkultur 3 bis 6 Monate, insbesondere 4 Monate in dem Bodenbereich dauert, um eine vorgezüchtete Ammenpflanze zu ergeben, wobei der Luftteil der vorgezüchteten Ammenpflanze nach der Vorkultur abgeschnitten wird,
(b) wobei mindestens ein Samen oder mindestens eine Pflanze der Baumspezies in den Bodenbereich nach der Vorkultur eingeführt wird,
wobei die Baumspezies eine Ektomykorrhizakolonisierung der Wurzeln vor der Vorkultur in dem Bodenbereich (C1) aufweist, wobei C1 durch einen Faktor reduziert wird, der 2 bis 5 beträgt, im Vergleich mit der Ektomykorrhizakolonisierung der Wurzeln der Baumspezies, die in einem Kontrollboden, der die Baumspezies umfasst, aufgrund des Wachstums der exotischen Spezies in dem Bodenbereich vor der Vorkultur erhalten wird,
wobei C1 0,1 % oder weniger als 0,1 % mindestens eines Ektomykorrhizapilzes umfasst, der für das Unterstützen des Wachstums der Baumspezies in dem Boden unerlässlich ist,
wobei die Ektomykorrhizakolonisierung der Wurzeln der Baumspezies nach der Vorkultur der Ammenpflanze und 5 Monate langer Züchtung der Baumspezies in dem Bodenbereich (C2) in einer Menge im Bereich von70 % bis 100 % gewonnen wird.
wobei C2 Folgendes umfasst:
mindestens einen ungünstigen Ektomykorrhizapilz, der in dem Bodenbereich vor der Vorkultur in einem relativen Verhältnis gleich oder geringer als 0,1 % vorliegt
und/oder mindestens einen gewonnenen unerlässlichen Ektomykorrhizapilz in einem relativen Verhältnis von mindestens 10 %,
wobei die Ammenpflanze *Leptolaena Bojeriana* ist, wobei die Baumspezies *Uapaca bojeri L.* ist und die exotische Spezies *Pinus patula* oder *Eucalyptus calmadulensis* ist.

2. Verwendung nach Anspruch 1, wobei nur ein Samen einer Ammenpflanze oder nur eine Ammenpflanze vorgezüchtet wird.

3. Verwendung nach Anspruch 1 oder 2, wobei die unerlässlichen Ektomykorrhizapilze zum Unterstützen des Wachstums von *Uapaca bojeri* Ektomykorrhizapilze der folgenden Typen sind: Russula earlei, Amanita sp., Telephoroid ectomycorrhizal sp. oder ungezüchteter Ektomykorrhiza-Homobasidiomycetes-Klon E2.

4. Verwendung nach Anspruch 3, wobei eine exotische Spezies *Eucalyptus calmadilensis* Dehn vorher in dem Bodenbereich gezüchtet worden ist, wobei die Ektomykorrhizapilze, die für das Wachstum des *Uapaca bojeri* ungünstig sind, Ektomykorrhizapilze der folgenden Typen sind: *Bondarcevomyces taxi, Russula exalbicans* oder *Xerocomus chrysenteron* und die unerlässlichen Ektomykorrhizapilze zum Unterstützen des Wachstums des *Uapaca bojeri* Ektomykorrhizapilze der folgenden Typen sind: Russula earlei, Amanita sp., Telephoroid ectomycorrhizal sp. oder ungezüchteter Ektomykorrhiza-Homobasidiomycetes-Klon E2.

5. Verwendung nach Anspruch 4, wobei die unerlässlichen Ektomykorrhizapilze, die in einem relativen Verhältnis von mindestens 10 % nach der Vorkultur und 5 Monate Kultur des *Uapaca bojeri* gewonnen werden, unter den folgenden Typen ausgewählt werden: *Russula earlei, Amanita "sp.,* Telephoroid ectomycorrhizal sp. oder ungezüchtetem Ektomykorrhiza-Homobasidiomycetes-Klon E2 und ungünstige Ektomykorrhizapilze in einem relativen Verhältnis gleich oder geringer als 0,1 % nach der Vorkultur und 5 Monaten langer Kultur des *Uapaca bajeri* unter den folgenden Typen ausgewählt werden: *Bondarcevomyces taxi, Russula exalbicans* oder *Xerocomus chrysenteron.*

6. Verwendung nach Anspruch 3, wobei eine exotische Spezies *Pinus patula* Schiede ex Schtdl. & Cham vorher in dem Bodenbereich gezüchtet worden ist, wobei die Ektomykorrhizapilze, die für das Wachstum des *Uapaca bojeri* ungünstig sind, Ektomykorrhizapilze der folgenden Typen sind: *Bondarcevomyces taxi, Russula exalbicans* oder *Xerocomus chrysenteron* und die unerlässlichen Ektomykorrhizapilze zum Unterstützen des Wachstums des *Uapaca bojeri* Ektomykorrhizapilze der folgenden Typen sind: Russula earlei, Amanita sp., Telephoroid ectomycorrhizal sp. oder ungezüchteter Ektomykorrhiza-Homobasidiomycetes-Klon E2.

7. Verwendung nach Anspruch 6, wobei die unerlässlichen Ektomykorrhizapilze, die in einem relativen Verhältnis von etwa mindestens 10 % nach 5 Monate langer Kultur des *Uapaca bojeri* gewonnen werden, unter den folgenden Typen ausgewählt werden: *Russula earlei* oder ungezüchtetem Ektomykorrhiza-Homobasidiomycetes-Klon E2 und ungünstige Ektomykorrhizapilze in einem relativen Verhältnis gleich oder geringer als 0,1 % nach der Vorkultur und 5 Monaten Kultur des *Uapaca bajeri* unter den folgenden Typen ausgewählt werden: *Bondarcevomyces taxi* oder *Russula exalbicans.*

8. Verfahren zum Unterstützen des Wachstums von mindestens einem Samen oder mindestens einer Pflanze einer Baumspezies in einem Bodenbereich, in dem eine exotische Spezies vorher als Vorkultur gezüchtet worden ist, und Modifizieren der Ektomykorrhizakolonisierung der Wurzeln der Baumspezies in dem Bodenbereich, insbesondere einem Bodenbereich, der einem Volumen von etwa 2,5 l aufweist, umfassend:
(1) einen Schritt des Vorzüchtens von mindestens einem Samen einer Ammenpflanze in einem Bodenbereich oder von mindestens einer Ammenpflanze in dem Bodenbereich, wobei die Vorkultur 3 bis 6 Monate, insbesondere 4 Monate in dem Bodenbereich dauert, um eine vorgezüchtete Ammenpflanze, zu ergeben, wobei der Luftteil der vorgezüchteten Ammenpflanze nach der Vorkultur abgeschnitten wird,
(2) einen Schritt des Einführens des mindestens einen Samens oder der mindestens einen Pflanze einer Baumspezies in den Bodenbereich,
wobei die Ektomykorrhizakolonisierung der Wurzeln der Baumspezies, die in dem Bodenbereich vor oder ohne die Vorkultur (C1) gezüchtet worden ist, durch einen Faktor reduziert wird, der 2 bis 5 beträgt, im Vergleich mit der Ektomykorrhizakolonisierung, die in einem Kontrollboden, der die Baumspezies umfasst, aufgrund des Wachstums einer exotischen Spezies in dem Bodenbereich vor der Vorkultur erhalten wird, wobei die die Ektomykorrhizakolonisierung (C1) Folgendes umfasst:
mindestens einen unerlässlichen Ektomykorrhizapilz zum Unterstützen des Wachstums der Baumspezies im dem Bodenbereich in einem relativen Verhältnis gleich oder geringer als 0,1;
wobei die Ektomykorrhizakolonisierung der Wurzeln der Baumspezies nach der Vorkultur der Ammenpflanze und 5 Monate langer Kultur der Baumspezies (C2), insbesondere in einem Boden, der ein Volumen von etwa 2,5, aufweist, in einer Menge im Bereich von etwa 70 % bis etwa 100 % gewonnen wird, wobei die Ektomykorrhizakolonisierung (2) Folgendes umfasst: in einem relativen Verhältnis gleich oder geringer als 0,1 % des mindestens einen ungünstigen Ektomykorrhizapiltes, der in dem Bodenbereich vor der Vorkultur vorliegt und/oder in einem relativen Verhältnis von mindestens 10 % von mindestens einem von dem unerlässlichen Ektomykorrhizapilz.
wobei die Ammenpflanze *Leptolaena Bojeriana* ist, die Baumspezies *Uapaca bojeri;* ist und die exotische Spezies *Pinus patula* oder *Eucalyptus calmadulensis* ist.

9. Verfahren nach Anspruch 8, wobei die unerlässlichen Ektomykorrhizapilze zum Unterstützen des Wachstums von *Uapaca bojeri* in einem Kontollboden vor der Vorkultur der Ammenpflanze Ektomykorrhizapilze der folgenden Typen sind: *Russula earlei, Amanita sp.,* Telephoroid ectomycorrhizal sp. oder ungezüchteter Ektomykorrhiza-Homobasidiomycetes-Klon E2.

10. Verwendung nach Anspruch 9, wobei eine exotische Spezies, insbesondere *Eucalyptus calmadulensis,* vorher in dem Bodenbereich vorgezüchtet worden ist, wobei die Ektomykorrhizapilze, die für das Wachstum des *Uapaca bojeri* ungünstig sind, Ektomykorrhizapilze der folgenden Typen sind: *Bondarcevomyces taxi, Russula exalbicans* oder *Xerocomus chrysenteron* und die unerlässlichen Ektomykorrhizapilze zum Unterstützen des Wachstums des *Uapaca bojeri* Ektomykorrhizapilze der folgenden Typen sind: *Russula earlei, Amanita sp.,* Telephoroid ectomycorrhizal sp. oder ungezüchteter Ektomykorrhiza-Homobasidiomycetes-Klon E2.

11. Verwendung nach Anspruch 10, wobei die unerlässlichen Ektomykorrhizapilze, die in einem relativen Verhältnis von mindestens 10 % nach 5 Monate langer Kultur des *Uapaca bojeri* gewonnen werden, unter den folgenden Typen ausgewählt werden: *Russula earlei, Amanita sp.,* Telephoroid ectomycorrhizal sp. oder ungezüchtetem Ektomykorrhiza-Homobasidiomycetes-Klon E2 und ungünstige Ektomykorrhizapilze in einem relativen Verhältnis gleich oder geringer als 0,1 % nach der Vorkultur und 5 Monaten langer Kultur des *Uapaca bajeri* unter den folgenden Typen ausgewählt werden: *Bondarcevomyces taxi, Russula exalbicans* oder *Xerocomus chrysenteron.*

12. Verwendung nach Anspruch 9, wobei eine exotische Spezies, insbesondere *Pinus patula,* vorher in dem Bodenbereich vorgezüchtet worden ist, wobei Ektomykorrhizapilze, die für das Wachstum des *Uapaca bojeri* ungünstig sind, Ektomykorrhizapilze der folgenden Typen sind: *Bondarcevomyces taxi, Russula exalbicans* oder *Xerocomus chrysenteron* und die unerlässlichen Ektomykorrhizapilze zum Unterstützen des Wachstums des *Uapaca bojeri* Ektomykorrhizapilze der folgenden Typen sind: *Russula earlei, Amanita sp.,* Telephoroid ectomycorrhizal sp. oder ungezüchteter Ektomykorrhiza-Homobasidiomycetes-Klon E2.

13. Verwendung nach Anspruch 12, wobei die unerlässlichen Ektomykorrhizapilze, die in einem relativen Verhältnis von mindestens 10 % nach 5 Monate langer Kultur des *Uapaca bojeri* gewonnen werden, unter den folgenden Typen ausgewählt werden: *Russula earlei,* oder ungezüchtetem Ektomykorrhiza-Homobasidiomycetes-Klon E2 und ungünstige Ektomykorrhizapilze in einem relativen Verhältnis gleich oder geringer als 0,1 % nach der Vorkultur und 5 Monaten Kultur des *Uapaca bajeri* unter den folgenden Typen ausgewählt werden: *Bondarcevomyces taxi* oder *Russula exalbicans.*

## Revendications

1. Utilisation d'au moins une graine de plante nourrice dans une surface de sol ou d'au moins une plante nourrice, dans une surface de sol, dans lequel une espèce exotique a été précédemment cultivée, comme une préculture, pour promouvoir la croissance d'au moins une graine ou d'au moins une plante d'une espèce d'arbre dans ladite surface de sol, en modifiant la colonisation ectomycorhizienne des racines de ladite espèce d'arbre dans ladite surface de sol,
dans laquelle
(a) ladite préculture dure de 3 à 6 mois dans ladite surface de sol, en particulier 4 mois, pour donner une plante nourrice précultivée, les parties aériennes de ladite plante nourrice précultivée étant sectionnées après la préculture,
(b) ladite au moins une graine ou au moins une plante de ladite espèce d'arbre est introduite dans ladite surface de sol après ladite préculture,
ladite espèce d'arbre ayant une colonisation ectomycorhizienne des racines avant la préculture, dans ladite surface de sol, (C1), C1 étant diminuée d'un facteur compris de 2 à 5 par comparaison avec la colonisation ectomycorhizienne des racines de ladite espèce d'arbre,obtenue dans un sol témoin comprenant ladite espèce d'arbre, due à la croissance de ladite espèce exotique dans ladite surface de sol avant ladite préculture,
C1 comprenant 0.1 % ou moins de 0.1 % d'au moins un champignon ectomycorhizien essentiel pour promouvoir la croissance des racines de laite espèce d'arbre dans lendit sol,
la colonisation ectomycorhizienne des racines de ladite espèce d'arbre après ladite préculture de ladite plante nourrice et 5 mois de culture de ladite espèce d'arbre dans ladite surface de sol, (C2), étant récupéré dans une gamme de 70% à 100%, C2 comprenant :
au moins un champignon ectomycorhizien défavorable, présent dans ladite surface de sol avant ladite préculture, dans un ratio relatif égal ou inférieur à 0.1%
et/ou au moins un champignon ectomycorhizien essentiel récupéré dans un ratio relatif d'au moins 10%,
dans laquelle ladite plante nurse est *Leptolaena bojeriana,* ladite espèce d'arbre est *Uapaca bojeri L.,* et ladite espèce exotique est *Pinus patula* ou *Eucalyptus calmadulensis.*

2. Utilisation selon la revendication 1, dans laquelle seule une graine de la plante nourrice ou seule une plante nourrice est précultivée.

3. Utilisation selon la revendication 1 ou 2, dans laquelle lesdits champignons ectomycorhiziens pour promouvoir la croissance de *Uapaca bojeri* sont des champignons ectomycorhiziens des types suivants : *Russula earlei, Amanita sp.,* Telephoroid ectomycorrhizal sp. et le clone E2 homobasidiomycète ectomycorhizien non cultivé.

4. Utilisation selon la revendication 3, dans laquelle une espèce exotique *Eucalyptus calmadulensis* Dehn a précédemment été cultivée dans ladite surface de sol, lesdits champignons ectomycorhiziens défavorables à la croissance dudit *Uapaca bojeri* sont des champignons ectomycorhiziens des types suivants : *Bondarcevomyces taxi, Russula exalbicans* et *Xerocomus chrysenteron* et lesdits champignons ectomycorhiziens essentiels pour promouvoir la croissance dudit *Uapaca bojeri* sont des champignons ectomycorhiziens des types suivants : *Russula earlei, Amanita sp.,* Telephoroid ectomycorrhizal sp. et le clone E2 homobasidiomycète ectomycorhizien non cultivé.

5. Utilisation selon la revendication 4, dans laquelle lesdits champignons ectomycorhiziens essentiels qui sont récupérés dans un ratio relatif d'au moins 10%, après ladite préculture et 5 mois de culture dudit *Uapaca bojeri* sont choisis parmi les types suivants : *Russula earlei, Amanita sp.,* Telephoroid ectomycorrhizal sp. et le clone E2 homobasidiomycète ectomycorhizien non cultivé, et les champignons ectomycorhiziens défavorables dans un ratio relatif égal ou inférieur à 0.1% après ladite préculture et 5 mois de culture dudit *Uapaca bojeri,* sont choisis parmi les types suivants : *Bondarcevomyces taxi, Russula exalbicans* et *Xerocomus chrysenteron.*

6. Utilisation selon la revendication 3, dans laquelle une espèce exotique *Pinus patula* Schiede ex Schtdl. & Cham a précédemment été cultivée dans ladite surface de sol, lesdits champignons ectomycorhiziens défavorables à la croissance dudit *Uapaca bojeri* sont de types suivants: *Bondarcevomyces taxi, Russula exalbicans* ou *Xerocomus chrysenteron,* et lesdits champignons ectomycorhiziens essentiels pour promouvoir la croissance dudit *Uapaca bojeri* sont des champignons ectomycorhiziens des types suivants : *Russula earlei, Amanita sp.,* Telephoroid ectomycorrhizal sp. et le clone E2 homobasidiomycète ectomycorhizien non cultivé.

7. Utilisation selon la revendication 6, dans laquelle lesdits champignons ectomycorhiziens essentiels qui sont récupérés dans un ratio d'environ au moins 10%, après 5 mois de culture dudit Uapaca bojeri sont choisis parmi les types suivants : *Russula earlei* et le clone E2 homobasidiomycète ectomycorhizien non cultivé, et les champignons ectomycorhiziens défavorables dans un ratio relatif égal ou inférieur à 0.1% après ladite préculture et 5 mois de culture dudit *Uapaca bojeri* sont choisis parmi les types suivants : *Bondarcevomyces taxi* et *Russula exalbicans.*

8. Procédé pour promouvoir la croissance d'au moins une graine ou d'au moins une plante d'une espèce d'arbre dans ladite surface de sol, dans laquelle une espèce exotique a précédemment été cultivée, et modifier la colonisation ectomycorhizienne des racines de ladite espèce d'arbre dans ladite surface de sol, en particulier une surface de sol ayant un volume approximatif de 2.5 L., comprenant :
(1) une étape de préculture d'au moins une graine d'une plante nourrice dans ladite surface de sol ou d'au moins une plante nourrice, dans ladite surface de sol, ladite préculture durant de 3 à 6 mois, en particulier 4 mois, pour donner une plante précultivée, les parties aériennes de ladite plante nourrice précultivée étant sectionnées après la préculture,
(2) une étape d'introduction de ladite au moins une graine ou de ladite au moins une plante d'une espèce d'arbre dans ladite surface de sol,
la colonisation ectomycorhizienne des racines de ladite espèce d'arbre cultivée dans ladite surface de sol, avant ou sans ladite préculture (C1), étant diminuée d'un facteur compris de 2 à 5, par comparaison avec la colonisation ectomycorhizienne obtenue dans un sol témoin comprenant ladite espèce d'arbre, en raison de la croissance d'une espèce exotique dans ladite surface de sol avant ladite préculture, ladite colonisation ectomycorhizienne (C1) comprenant :
au moins un champignon ectomycorhizien essentiel pour promouvoir la croissance de ladite espèce d'arbre dans ladite surface de sol dans un ratio relatif égal ou inférieur à 0.1 % ;
la colonization ectomycorhizienne des racines de ladite espèce d'arbre, après ladite préculture de ladite plante nourrice et 5 mois de culture de ladite espèce d'arbre (C2), en particulier dans une surface de sol ayant un volume approximatif de 2.5 L, étant récupérée dans une gamme d'environ 70% à environ 100%, ladite colonisation ectomycorhizienne (C2) comprenant :
dans un ratio relatif, égal ou inférieur à 0.1% dudit au moins un champignon ectomycorhizien défavorable, présent dans ladite surface de sol avant ladite préculture, et/ou dans un ratio relatif, au moins 10%, d'au moins un champignon ectomycorhizien essentiel récupéré,
dans lequel ladite plante nourrice est *Leptolaena bojeriana,* ladite espèce d'arbre est *Uapaca bojeri L.,* et ladite espèce exotique est *Pinus patula* ou *Eucalyptus calmadulensis.*

9. Procédé selon la revendication 10, dans lequel lesdits champignons ectomycorhiziens essentiels pour promouvoir la croissance de *Uapaca bojeri* dans un sol contrôle avant la préculture de ladite plante nourrice sont des champignons ectomycorhiziens des types suivants : *Russula earlei, Amanita sp.,* Telephoroid ectomycorrhizal sp. et le clone E2 homobasidiomycète ectomycorhizien non cultivé.

10. Procédé selon la revendication 9, dans lequel une espèce exotique a été précédemment cultivée dans ladite surface de sol, en particulier *Eucaliptus calmadulensis,* lesdits champignons ectomycorhiziens défavorables à la croissance dudit *Uapaca bojeri* sont des champignons ectomycorhiziens des types suivants : *Bondarcevomyces taxi, Russula exalbicans* et *Xerocomus chrysenteron,* et lesdits champignons ectomycorhiziens essentiels pour promouvoir la croissance dudit *Uapaca bojeri* sont des champignons ectomycorhiziens des types suivants : *Russula earlei, Amanita sp.,* Telephoroid ectomycorrhizal sp. et le clone E2 homobasidiomycète ectomycorhizien non cultivé.

11. Procédé selon la revendication 10, dans lequel lesdits champignons ectomycorhiziens essentiels qui sont récupérés dans un ratio relatif d'au moins 10%, après ladite préculture et 5 mois de culture dudit *Uapaca bojeri* sont choisis parmi les types suivants : *Russula earlei, Amanita sp.,* Telephoroid ectomycorrhizal sp. et le clone E2 homobasidiomycète ectomycorhizien non cultivé, et les champignons ectomycorhiziens défavorables dans un ratio relatif égal ou inférieur à 0.1% après ladite préculture et 5 mois de culture dudit *Uapaca bojeri* sont choisis parmi les types suivants: *Bondarcevomyces taxi, Russula exalbicans et Xerocomus chrysenteron.*

12. Procédé selon la revendication 9, dans lequel une espèce exotique a précédemment été cultivée dans ladite surface de sol, en particulier *Pinus patula,* lesdits champignons ectomycorhiziens défavorables pour la croissance dudit *Uapaca bojeri* sont des champignons ectomycorhiziens des types suivants : *Bondarcevomyces taxi, Russula exalbicans* et *Xerocomus chrysenteron,* et les champignons ectomycorhiziens essentiels pour promouvoir la croissance dudit *Uapaca bojeri* sont des champignons ectomycorhiziens des types suivants : *Russula earlei, Amanita sp.,* Telephoroid ectomycorrhizal sp. et le clone E2 homobasidiomycète ectomycorhizien non cultivé.

13. Procédé selon la revendication 12, dans lequel lesdits champignons ectomycorhiziens essentiels qui sont récupérés dans un ratio relatif d'au moins 10%, après 5 mois de culture dudit *Uapaca bojeri* sont choisis parmi les types suivants : *Russula earlei,* et le clone E2 homobasidiomycète ectomycorhizien non cultivé, et les champignons ectomycorhiziens défavorables dans un ratio relatif égal ou inférieur à 0.1 % après ladite préculture et 5 mois de culture dudit *Uapaca bojeri* sont choisis parmi les types suivants : *Bondarcevomyces taxi* et *Russula exalbicans.*
